(19) Europäisches Patentamt
European Patent Office
Office européen des brevets

(11) Veröffentlichungsnummer: **0 413 221 A2**

# (12) EUROPÄISCHE PATENTANMELDUNG

(21) Anmeldenummer: **90115018.5**

(22) Anmeldetag: **04.08.90**

(51) Int. Cl.5: **A01N 47/42**, C07D 251/46, C07D 251/42, C07D 239/42, C07D 239/47, C07D 239/52, C07D 401/12

(30) Priorität: **17.08.89 DE 3927140**

(43) Veröffentlichungstag der Anmeldung: **20.02.91 Patentblatt 91/08**

(84) Benannte Vertragsstaaten: **BE CH DE FR GB IT LI NL**

(71) Anmelder: **BAYER AG**

**D-5090 Leverkusen 1 Bayerwerk(DE)**

(72) Erfinder: **Gesing, Ernst R., Dr.**
**Trillser Graben 4**
**D-4006 Erkrath-Hochdahl(DE)**
Erfinder: **Fest, Christa Dr.**
**Im Johannistal 20**
**D-5600 Wuppertal 1(DE)**
Erfinder: **Kirsten, Rolf, Dr.**
**Carl-Langhans-Strasse 27**
**D-4019 Monheim(DE)**
Erfinder: **Kluth, Joachim, Dr.**
**Tannenweg 9**
**D-4018 Langenfeld 9(DE)**
Erfinder: **Müller, Klaus-Helmut, Dr.**
**Bockhackstrasse 55**
**D-4000 Düsseldorf 13(DE)**
Erfinder: **Riebel, Hans-Jochem, Dr.**
**In der Beek 92**
**D-5600 Wuppertal 1(DE)**
Erfinder: **Santel, Hans-Joachim, Dr.**
**Grünstrasse 9a**
**D-5090 Leverkusen 1(DE)**
Erfinder: **Lürssen, Klaus, Dr.**
**August-Kierspel-Strasse 145**
**D-5060 Bergisch Gladbach 2(DE)**
Erfinder: **Schmidt, Robert R., Dr.**
**Im Waldwinkel 110**
**D-5060 Bergisch Gladbach 2(DE)**

## (54) Selektive Herbizide auf Basis von Sulfonylisothioharnstoffen.

(57) Die Erfindung betrifft die Verwendung von teilweise bekannten Sulfonylisothioharnstoffen der allgemeinen Formel (I),

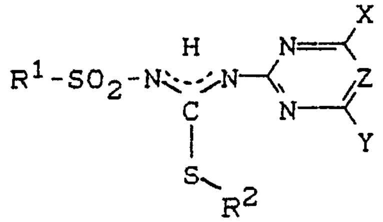

(I)

in welcher
$R^1$ für jeweils gegebenenfalls substiutiertes, Phenyl, Pyridyl, Benzyl, Pyridylmethyl, Thienyl, Pyrazolyl oder Naphthyl steht,
$R^2$ für jeweils gegebenenfalls substiutiertes $C_1$-$C_4$-Alkyl, Benzyl oder Phenylethyl steht,
Z für N oder C-$R^3$ steht, wobei die Substituenten X, Y und $R^3$ die in der Beschreibung angegebene Bedeutung haben,

EP 0 413 221 A2

als selektive Herbizide in verschiedenen Kulturpflanzen; die Wirkstoffe der Formel (I) eignen sich insbesondere zur selektiven Bekämpfung von dikotylen Unkräutern in monokotylen Kulturen, wie z.B. Weizen, im Vorauflauf- und Nachauflaufverfahren.
Eine bestimmte, definierte Untergruppe von Verbindungen der Formel (I) ist neu und ist ebenfalls Gegenstand der vorliegenden Erfindung.

# SELEKTIVE HERBIZIDE AUF BASIS VAN SULFONYLISOTHIOHARNSTOFFEN

Die vorliegende Erfindung betrifft die Verwendung von teilweise bekannten Sulfonylisothioharnstoffen als selektive Herbizide sowie bestimmte neue Sulfonylisothioharnstoffe.

Verschiedene Sulfonylisothioharnstoffe sind als potentielle Herbizide bekannt geworden, haben jedoch bisher als Mittel zur Unkrautbekämpfung keine praktische Bedeutung erlangt (vgl. EP-A 5986, EP-A 23141, EP-A 30142 und DE-OS 3618004/LeA 24460).

Es wurde nun gefunden, daß Sulfonylisothioharnstoffe der allgemeinen Formel (I)

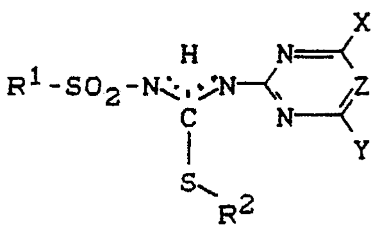

(I)

in welcher

$R^1$ für Phenyl oder Pyridyl steht, welche jeweils 1 bis 3, gleiche oder verschiedene Substituenten aus der Reihe Halogen, Cyano, $C_1$-$C_4$-Alkyl, $C_1$-$C_4$-Halogenalkyl, $C_1$-$C_4$-Alkoxy, $C_1$-$C_4$-Alkylthio, $C_1$-$C_4$-Alkylsulfinyl, $C_1$-$C_4$-Alkylsulfonyl, Di-($C_1$-$C_4$-alkyl)-amino-sulfonyl, N-($C_1$-$C_4$-Alkoxy)-N-($C_1$-$C_4$-alkyl)-amino-sulfonyl, Phenyl, Phenylsulfonyl, $C_1$-$C_6$-Alkoxy-carbonyl (welches gegebenenfalls durch Halogen oder $C_1$-$C_4$-Alkoxy substituiert ist) enthalten, oder

$R^1$ für Benzyl oder Pyridylmethyl steht, welche jeweils 1 bis 3, gleiche oder verschiedene Substituenten aus der Reihe Halogen, Cyano, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkoxy-carbonyl, Di-($C_1$-$C_4$-alkyl)-amino-carbonyl enthalten, oder

$R^1$ für Thienyl oder Pyrazolyl steht, welche jeweils 1 bis 3, gleiche oder verschiedene Substituenten aus der Reihe Halogen, $C_1$-$C_4$-Alkyl, $C_1$-$C_4$-Halogenalkyl, $C_1$-$C_4$-Alkaxy-carbonyl enthalten, oder

$R^1$ für Naphthyl steht,

$R^2$ für $C_1$-$C_4$-Alkyl (welches gegebenenfalls durch Halogen, Cyano, $C_1$-$C_4$-Alkoxy, Carboxy, $C_1$-$C_4$-Alkoxycarbonyl, Aminocarbonyl, $C_1$-$C_4$-Alkylamino-carbonyl oder Di-($C_1$-$C_4$-alkyl)-amino-carbonyl substituiert ist), für Benzyl oder für Phenylethyl steht (welche jeweils gegebenenfalls durch Halogen, $C_1$-$C_4$-Alkyl, $C_1$-$C_4$-Alkoxy oder $C_1$-$C_4$-Alkoxy-carbonyl substituiert sind),

X für Wasserstoff, Halogen, $C_1$-$C_4$-Alkyl, $C_1$-$C_4$-Halogenalkyl, Cyclopropyl, $C_1$-$C_4$-Alkoxy, $C_1$-$C_4$-Halogenalkoxy, $C_1$-$C_4$-Alkylthio, $C_1$-$C_4$-Alkylamino oder Di-($C_1$-$C_4$-alkyl)-amino steht,

Y für $C_1$-$C_4$-Alkyl, $C_1$-$C_4$-Halogenalkyl, Cyclopropyl, $C_1$-$C_4$-Alkoxy oder $C_1$-$C_4$-Halogenalkoxy steht und

Z für Stickstoff oder eine C-$R^3$-Gruppierung steht,

worin

$R^3$ für Wasserstoff, Halogen, $C_1$-$C_4$-Alkyl, $C_1$-$C_4$-Alkoxy, $C_1$-$C_4$-Halogenalkoxy oder $C_1$-$C_4$-Alkoxy-$C_1$-$C_4$-alkoxy steht,

sehr gut zur selektiven Bekämpfung von Unkräutern in Kulturpflanzen geeignet sind.

Die erfindungsgemäß zu verwendenden Sulfonylisothioharnstoffe sind durch die Formel (I) allgemein definiert. Vorzugsweise stehen in Formel (I)

$R^1$ für Phenyl oder Pyridyl, welche jeweils 1 oder 2, gleiche oder verschiedene Substituenten aus der Reihe Fluor, Chlor, Brom, Cyano, Methyl, Trifluormethyl, Methoxy, Ethoxy, Methylthio, Ethylthio, Propylthio, Isopropylthio, Methylsulfinyl, Ethylsulfinyl, Methylsulfonyl, Ethylsulfonyl, Dimethyl aminosulfonyl, Diethylaminosulfonyl, N-Methoxy-N-methyl-amino-sulfonyl, Phenyl, Phenylsulfonyl, Methoxycarbonyl, Ethoxycarbonyl, Propoxycarbonyl, Isopropoxycarbonyl, 2-Chlor-ethoxy-carbonyl, 2-Methoxy-ethoxy-carbonyl, 2-Ethoxyethoxy-carbonyl enthalten, oder

$R^1$ für Benzyl oder Pyridylmethyl, welche jeweils 1 oder 2, gleiche oder verschiedene Substituenten aus der Reihe Fluor, Chlor, Brom, Methyl, Trifluormethyl, Methoxy, Difluormethoxy, Trifluormethoxy, Methoxycarbonyl, Ethoxycarbonyl, Dimethylaminocarbonyl enthalten, oder

$R^1$ für Thienyl oder Pyrazolyl, welche jeweils 1 oder 2, gleiche oder verschiedene Substituenten aus der Reihe Fluor, Chlor, Brom, Methyl, Ethyl, Trifluormethyl, Methoxycarbonyl, Ethoxycarbonyl enthalten, oder für Naphthyl,

$R^2$ für Methyl, Ethyl, Cyanomethyl, Carboxymethyl, Methoxycarbonylmethyl, Ethoxycarbonylmethyl oder Benzyl,
X für Wasserstoff, Chlor, Methyl, Ethyl, Trifluormethyl, Cyclopropyl, Methoxy, Ethoxy, Difluormethoxy, Methylthio, Methylamino oder Dimethylamino,
Y für Methyl, Ethyl, Trifluormethyl, Cyclopropyl, Methoxy, Ethoxy oder Difluormethoxy und
Z für Stickstoff oder eine C-$R^3$-Gruppierung,
worin
$R^3$ für Wasserstoff, Chlor oder Methyl steht.

Eine besonders bevorzugte Gruppe von Verbindungen der Formel (I) sind diejenigen, bei denen $R^1$, $R^2$, X und Y die oben als bevorzugt angegebenen Bedeutungen haben und Z für Stickstoff steht.

Eine weitere besonders bevorzugte Gruppe von Verbindungen der Formel (I) sind diejenigen, bei denen $R^1$, $R^2$, X und Y die oben als bevorzugt angegebenen Bedeutungen haben und Z für eine CH-Gruppierung steht.

Beispiele für die erfindungsgemäß zu verwendenden Verbindungen der Formel (I) sind in der nachstehenden Tabelle 1 aufgeführt.

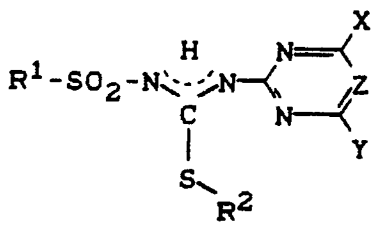

(I)

<u>Tabelle 1:</u> Beispiele für die Verbindungen der Formel (I)

| Bsp.-Nr. | $R^1$ | $R^2$ | X | Y | Z | Schmelzpunkt (°C) |
|---|---|---|---|---|---|---|
| 1 | 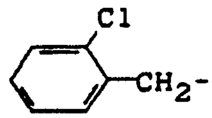 | $CH_3$ | $CH_3$ | $CH_3$ | N | 179 |
| 2 | 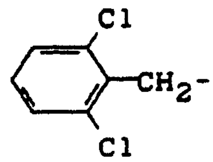 | $CH_3$ | $CH_3$ | $CH_3$ | N | 204 |
| 3 | 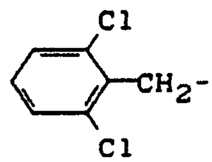 | $CH_3$ | $CH_3$ | $CH_3$ | CH | 193 |
| 4 | 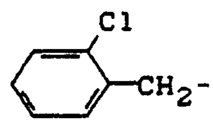 | $CH_3$ | $CH_3$ | $CH_3$ | CH | 170 |
| 5 | 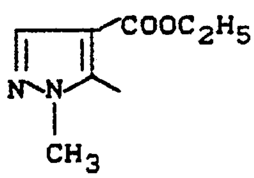 | $CH_3$ | $CH_3$ | $CH_3$ | CH | 130 |
| 6 | 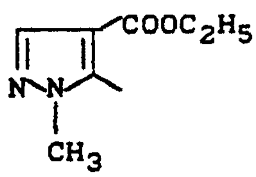 | $CH_3$ | $CH_3$ | $CH_3$ | N | 151 |
| 7 | 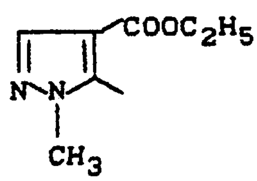 | $CH_3$ | $OCH_3$ | $OCH_3$ | N | 142 |

Tabelle 1: Beispiele für die Verbindungen der Formel (I)

| Bsp.-Nr. | $R^1$ | $R^2$ | X | Y | Z | Schmelzpunkt (°C) |
|---|---|---|---|---|---|---|
| 8 | 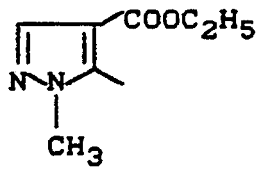 | $CH_3$ | $N(CH_3)_2$ | $CH_3$ | N | 113 |
| 9 | 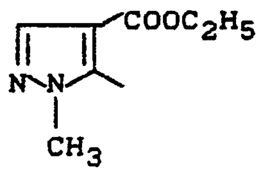 | $CH_3$ | $OCH_3$ | $OCH_3$ | CH | 117 |
| 10 | 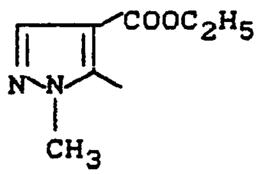 | $CH_3$ | $SCH_3$ | $CH_3$ | N | 125 |
| 11 | 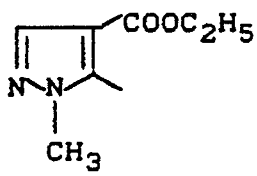 | $CH_3$ | $CH_3$ | $OCH_3$ | N | 120 |
| 12 | 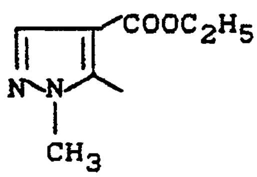 | $CH_3$ | $CH_3$ | $OCH_3$ | CH | 127 |
| 13 | 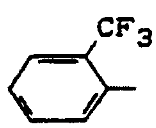 | $CH_3$ | $OCH_3$ | $OCH_3$ | N | 152 |
| 14 | 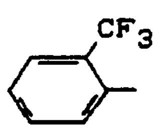 | $CH_3$ | $SCH_3$ | $CH_3$ | N | 136 |

Tabelle 1: Beispiele für die Verbindungen der Formel (I)

| Bsp.-Nr. | $R^1$ | $R^2$ | X | Y | Z | Schmelzpunkt (°C) |
|---|---|---|---|---|---|---|
| 15 | 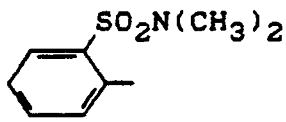 | $CH_3$ | $CH_3$ | $OCH_3$ | N | 153 |
| 16 | 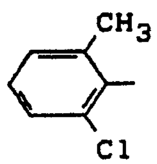 | $CH_3$ | $OCH_3$ | $CH_3$ | N | 191 |
| 17 | 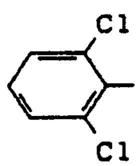 | $CH_3$ | $CH_3$ | $OCH_3$ | N | 155 |
| 18 | 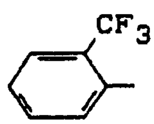 | $CH_3$ | $CH_3$ | $OCH_3$ | N | 205 |
| 19 | 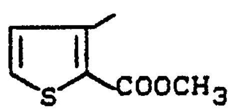 | $CH_3$ | $SCH_3$ | $CH_3$ | N | 162 |
| 20 | 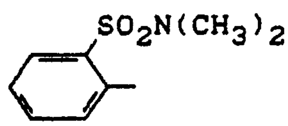 | $CH_3$ | $SCH_3$ | $CH_3$ | N | 148 |
| 21 | 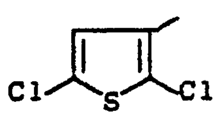 | $CH_3$ | $SCH_3$ | $CH_3$ | N | 156 |
| 22 | 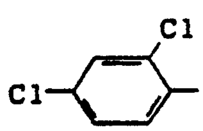 | $CH_3$ | $CH_3$ | $OCH_3$ | N | 148 |

Tabelle 1: Beispiele für die Verbindungen der Formel (I)

| Bsp.-Nr. | $R^1$ | $R^2$ | X | Y | Z | Schmelzpunkt ($^\circ C$) |
|---|---|---|---|---|---|---|
| 23 | 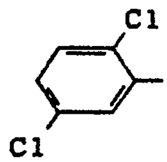 | $CH_3$ | $CH_3$ | $OCH_3$ | N | 206 |
| 24 | 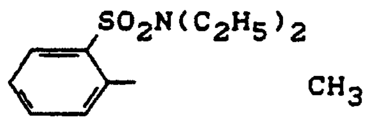 | $CH_3$ | $CH_3$ | $OCH_3$ | N | 138 |
| 25 | 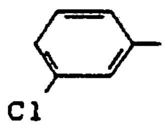 | $CH_3$ | $CH_3$ | $OCH_3$ | N | 149 |
| 26 | 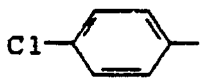 | $CH_3$ | $CH_3$ | $OCH_3$ | N | 193 |
| 27 | 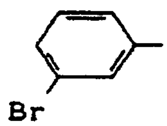 | $CH_3$ | $CH_3$ | $OCH_3$ | N | 153 |
| 28 | 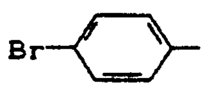 | $CH_3$ | $CH_3$ | $OCH_3$ | N | 190 |
| 29 | 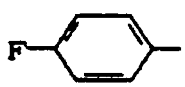 | $CH_3$ | $CH_3$ | $OCH_3$ | N | 174 |
| 30 | 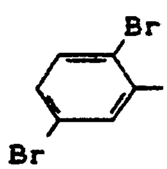 | $CH_3$ | $CH_3$ | $OCH_3$ | N | 167 |

Tabelle 1: Beispiele für die Verbindungen der Formel (I)

| Bsp.-Nr. | $R^1$ | $R^2$ | X | Y | Z | Schmelzpunkt (°C) |
|---|---|---|---|---|---|---|
| 31 | 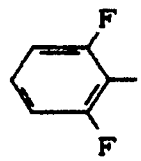 | $CH_3$ | $CH_3$ | $OCH_3$ | N | 163 |
| 32 | 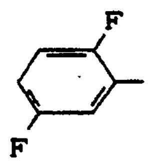 | $CH_3$ | $CH_3$ | $OCH_3$ | N | 109 |
| 33 | 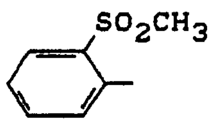 | $CH_3$ | $CH_3$ | $OCH_3$ | N | 175 |
| 34 | 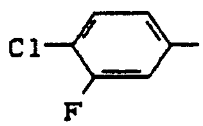 | $CH_3$ | $CH_3$ | $OCH_3$ | N | 151 |
| 35 | 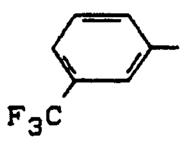 | $CH_3$ | $CH_3$ | $OCH_3$ | N | 133 |
| 36 | 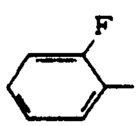 | $CH_3$ | $OCH_3$ | $OCH_3$ | N | 143 |
| 37 | 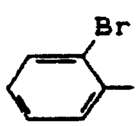 | $CH_3$ | $OCH_3$ | $OCH_3$ | N | 150 |
| 38 | 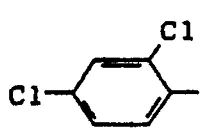 | $CH_3$ | $OCH_3$ | $OCH_3$ | N | 170 |

Tabelle 1: Beispiele für die Verbindungen der Formel (I)

| Bsp.-Nr. | $R^1$ | $R^2$ | X | Y | Z | Schmelzpunkt (°C) |
|---|---|---|---|---|---|---|
| 39 | 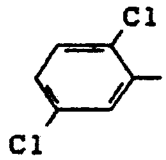 | $CH_3$ | $OCH_3$ | $OCH_3$ | N | 185 |
| 40 | 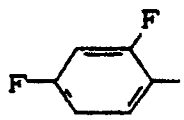 | $CH_3$ | $OCH_3$ | $OCH_3$ | N | 165 |
| 41 | 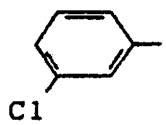 | $CH_3$ | $OCH_3$ | $OCH_3$ | N | 140 |
| 42 | 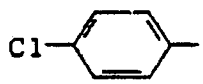 | $CH_3$ | $OCH_3$ | $OCH_3$ | N | 182 |
| 43 | 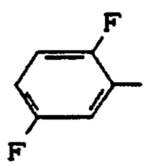 | $CH_3$ | $OCH_3$ | $OCH_3$ | N | 113 |
| 44 | 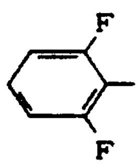 | $CH_3$ | $OCH_3$ | $OCH_3$ | N | 151 |
| 45 | 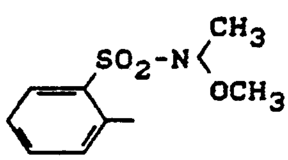 | $CH_3$ | $CH_3$ | $CH_3$ | N | 180 |

<u>Tabelle 1:</u> Beispiele für die Verbindungen der Formel (I)

| Bsp.-Nr. | $R^1$ | $R^2$ | X | Y | Z | Schmelzpunkt (°C) |
|---|---|---|---|---|---|---|
| 46 | 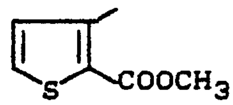 | $CH_3$ | $CH_3$ | $OCH_3$ | N | 173 |
| 47 | 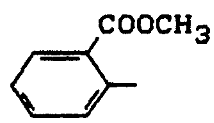 | $CH_3$ | $CH_3$ | $OCH_3$ | N | 155 |
| 48 | 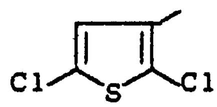 | $CH_3$ | $CH_3$ | $OCH_3$ | N | 153 |
| 49 | 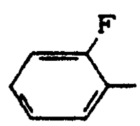 | $CH_3$ | $CH_3$ | $OCH_3$ | N | 126 |
| 50 | 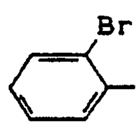 | $CH_3$ | $CH_3$ | $OCH_3$ | N | 145 |
| 51 | 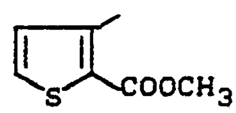 | $CH_3$ | $CH_3$ | $CH_3$ | N | 175 |
| 52 | 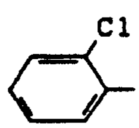 | $CH_3$ | $CH_3$ | $CH_3$ | N | 153 |
| 53 | 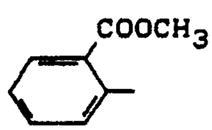 | $CH_3$ | $CH_3$ | $CH_3$ | N | 134 |

Tabelle 1: Beispiele für die Verbindungen der Formel (I)

| Bsp.-Nr. | $R^1$ | $R^2$ | X | Y | Z | Schmelzpunkt (°C) |
|---|---|---|---|---|---|---|
| 54 | 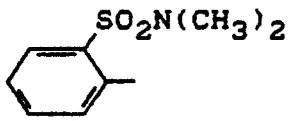 | $CH_3$ | $CH_3$ | $CH_3$ | N | 156 |
| 55 | 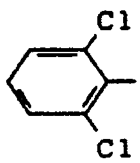 | $CH_3$ | $CH_3$ | $CH_3$ | N | 170 |
| 56 | 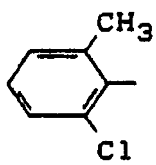 | $CH_3$ | $CH_3$ | $CH_3$ | N | 173 |
| 57 | 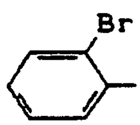 | $CH_3$ | $CH_3$ | $CH_3$ | N | 175 |
| 58 | 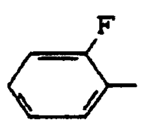 | $CH_3$ | $CH_3$ | $CH_3$ | N | 159 |
| 59 | 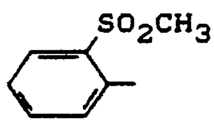 | $CH_3$ | $CH_3$ | $CH_3$ | N | 152 |
| 60 | 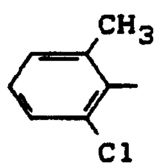 | $CH_3$ | $OCH_3$ | $OCH_3$ | N | 165 |
| 61 | 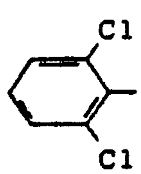 | $CH_3$ | $OCH_3$ | $OCH_3$ | N | 185 |

<u>Tabelle 1:</u> Beispiele für die Verbindungen der Formel (I)

| Bsp.-Nr. | $R^1$ | $R^2$ | X | Y | Z | Schmelzpunkt (°C) |
|---|---|---|---|---|---|---|
| 62 | 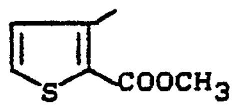 | $CH_3$ | $OCH_3$ | $OCH_3$ | N | 194 |
| 63 | 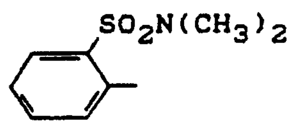 | $CH_3$ | $OCH_3$ | $OCH_3$ | N | 167 |
| 64 | 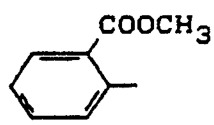 | $CH_3$ | $SCH_3$ | $CH_3$ | N | 156 |
| 65 | 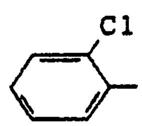 | $CH_3$ | $SCH_3$ | $CH_3$ | N | 132 |
| 66 | 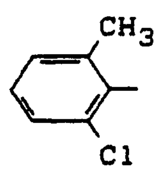 | $CH_3$ | $SCH_3$ | $CH_3$ | N | 195 |
| 67 | 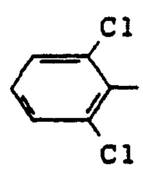 | $CH_3$ | $SCH_3$ | $CH_3$ | N | 203 |
| 68 | 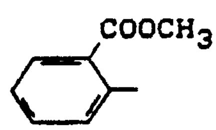 | $CH_3$ | $CH_3$ | $OC_2H_5$ | N | 92 |
| 69 | 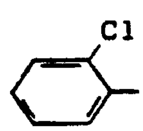 | $CH_3$ | $CH_3$ | $OC_2H_5$ | N | 125 |

Tabelle 1: Beispiele für die Verbindungen der Formel (I)

| Bsp.-Nr. | $R^1$ | $R^2$ | X | Y | Z | Schmelzpunkt (°C) |
|---|---|---|---|---|---|---|
| 70 | 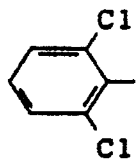 | $CH_3$ | $CH_3$ | $OC_2H_5$ | N | 229 |
| 71 | 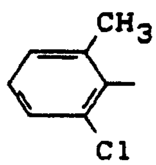 | $CH_3$ | $CH_3$ | $OC_2H_5$ | N | 209 |
| 72 | 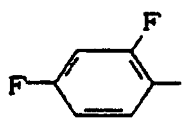 | $CH_3$ | $CH_3$ | $OCH_3$ | N | 152 |
| 73 | 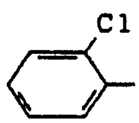 | $CH_3$ | $OCH_3$ | $OCH_3$ | N | 181 |
| 74 | 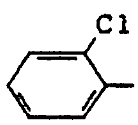 | $CH_3$ | $C_2H_5$ | $OCH_3$ | N | 132 |
| 75 | 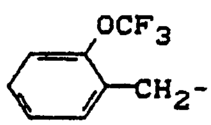 | $CH_3$ | $OCH_3$ | $OCH_3$ | N | 166 |
| 76 | 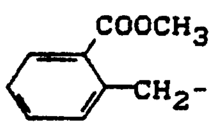 | $CH_3$ | $SCH_3$ | $CH_3$ | N | 153 |
| 77 | 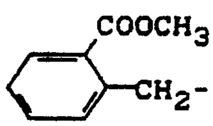 | $CH_3$ | $CH_3$ | $OC_2H_5$ | N | 134 |

<u>Tabelle 1:</u> Beispiele für die Verbindungen der Formel (I)

| Bsp.-Nr. | $R^1$ | $R^2$ | X | Y | Z | Schmelzpunkt (°C) |
|---|---|---|---|---|---|---|
| 78 | 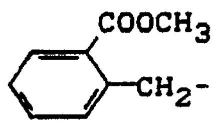 | $CH_3$ | $N(CH_3)_2$ | $CH_3$ | N | 184 |
| 79 | 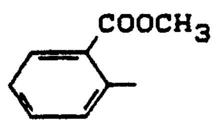 | $CH_3$ | $C_2H_5$ | $OCH_3$ | N | 123 |
| 80 | 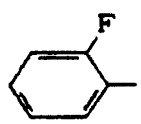 | $CH_3$ | $C_2H_5$ | $OCH_3$ | N | 118 |
| 81 | 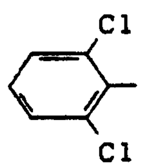 | $CH_3$ | $C_2H_5$ | $OCH_3$ | N | 129 |
| 82 | 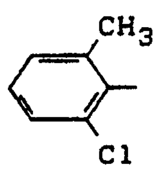 | $CH_3$ | $C_2H_5$ | $OCH_3$ | N | 122 |
| 83 | 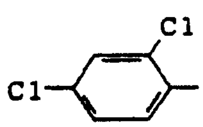 | $CH_3$ | $C_2H_5$ | $OCH_3$ | N | 152 |
| 84 | 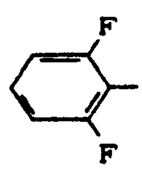 | $CH_3$ | $C_2H_5$ | $OCH_3$ | N | 112 |
| 85 | 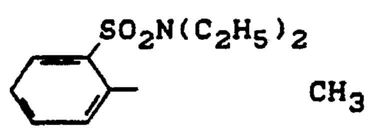 | $CH_3$ | $C_2H_5$ | $OCH_3$ | N | 124 |

<u>Tabelle 1:</u> Beispiele für die Verbindungen der Formel (I)

| Bsp.-Nr. | $R^1$ | $R^2$ | X | Y | Z | Schmelzpunkt (°C) |
|---|---|---|---|---|---|---|
| 86 | 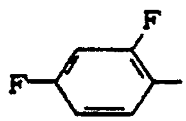 | $CH_3$ | $C_2H_5$ | $OCH_3$ | N | 144 |
| 87 | 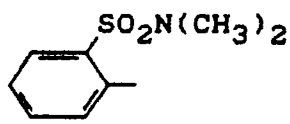 | $CH_3$ | $C_2H_5$ | $OCH_3$ | N | 150 |
| 88 | 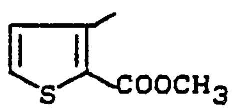 | $CH_3$ | $C_2H_5$ | $OCH_3$ | N | 116 |
| 89 | 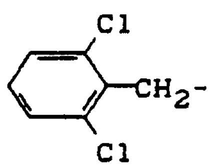 | $CH_3$ | $OCH_3$ | $OCH_3$ | N | 224 |
| 90 | 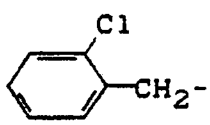 | $CH_3$ | $OCH_3$ | $OCH_3$ | N | 164 |
| 91 | 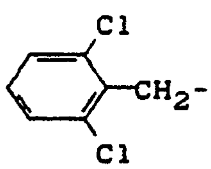 | $CH_3$ | $CH_3$ | $OCH_3$ | N | 219 |
| 92 | 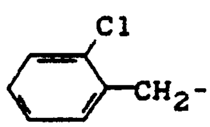 | $CH_3$ | $CH_3$ | $OCH_3$ | N | 197 |
| 93 | 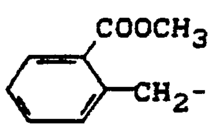 | $CH_3$ | $OCH_3$ | $OCH_3$ | N | 161 |

Tabelle 1: Beispiele für die Verbindungen der Formel (I)

| Bsp.-Nr. | $R^1$ | $R^2$ | X | Y | Z | Schmelzpunkt (°C) |
|---|---|---|---|---|---|---|
| 94 | 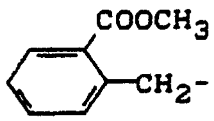 | $CH_3$ | $CH_3$ | $OCH_3$ | N | 177 |
| 95 | 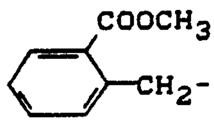 | $CH_3$ | $C_2H_5$ | $OCH_3$ | N | 140 |
| 96 | 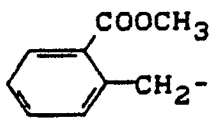 | $CH_3$ | $CH_3$ | $CH_3$ | N | 175 |
| 97 | 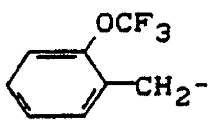 | $CH_3$ | $CH_3$ | $OCH_3$ | N | 169 |
| 98 | 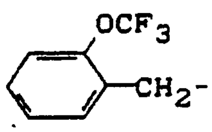 | $CH_3$ | $CH_3$ | $CH_3$ | N | 187 |
| 99 | 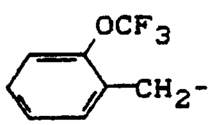 | $CH_3$ | $C_2H_5$ | $OCH_3$ | N | 151 |
| 100 | 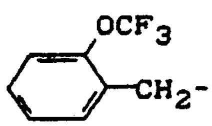 | $CH_3$ | $CH_3$ | $OC_2H_5$ | N | 158 |
| 101 | 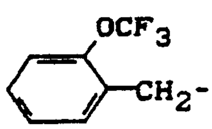 | $CH_3$ | $SCH_3$ | $CH_3$ | N | 178 |

Tabelle 1: Beispiele für die Verbindungen der Formel (I)

| Bsp.-Nr. | $R^1$ | $R^2$ | X | Y | Z | Schmelzpunkt (°C) |
|---|---|---|---|---|---|---|
| 102 | 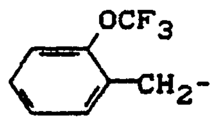 | $CH_3$ | $OCH_3$ | $OCH_3$ | N | 167 |
| 103 | 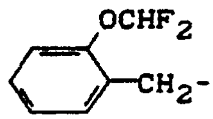 | $CH_3$ | $CH_3$ | $OCH_3$ | N | 172 |
| 104 | 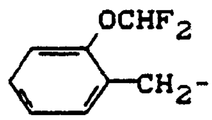 | $CH_3$ | $CH_3$ | $CH_3$ | N | 160 |
| 105 | 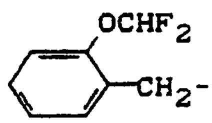 | $CH_3$ | $C_2H_5$ | $OCH_3$ | N | 114 |
| 106 | 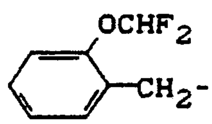 | $CH_3$ | $SCH_3$ | $CH_3$ | N | 162 |
| 107 | 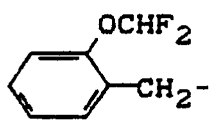 | $CH_3$ | $CH_3$ | $OC_2H_5$ | N | 160 |
| 108 | 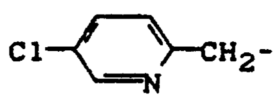 | $CH_3$ | $OCH_3$ | $OCH_3$ | N | 170 |
| 109 | 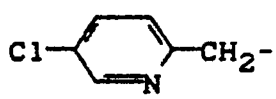 | $CH_3$ | $CH_3$ | $OCH_3$ | N | 118 |
| 110 | 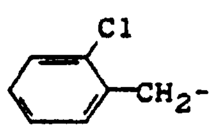 | $CH_3$ | $CH_3$ | $OC_2H_5$ | N | 157 |

**Tabelle 1:** Beispiele für die Verbindungen der Formel (I)

| Bsp.-Nr. | $R^1$ | $R^2$ | X | Y | Z | Schmelz-punkt (°C) |
|---|---|---|---|---|---|---|
| 111 | 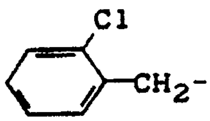 | $CH_3$ | $SCH_3$ | $CH_3$ | N | 194 |
| 112 | 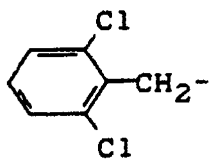 | $CH_3$ | $CH_3$ | $OC_2H_5$ | N | 160 |
| 113 | 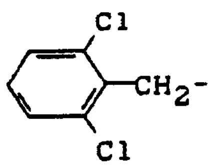 | $CH_3$ | $SCH_3$ | $CH_3$ | N | 147 |
| 114 | 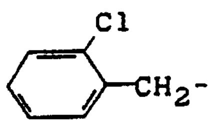 | $CH_3$ | $C_2H_5$ | $OCH_3$ | N | 127 |
| 115 | 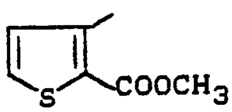 | $CH_3$ | $CH_3$ | $CH_3$ | CH | 199 |
| 116 | 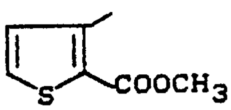 | $CH_3$ | $OCH_3$ | $OCH_3$ | CH | 210 |
| 117 | 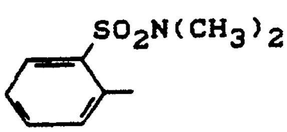 | $CH_3$ | $CH_3$ | $CH_3$ | CH | 181 |
| 118 | 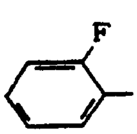 | $CH_3$ | $CH_3$ | $CH_3$ | CH | 165 |

Tabelle 1: Beispiele für die Verbindungen der Formel (I)

| Bsp.-Nr. | $R^1$ | $R^2$ | X | Y | Z | Schmelzpunkt (°C) |
|---|---|---|---|---|---|---|
| 119 | 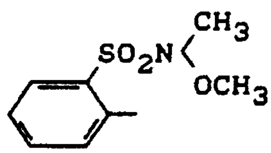 | $CH_3$ | $CH_3$ | $CH_3$ | CH | 180 |
| 120 | 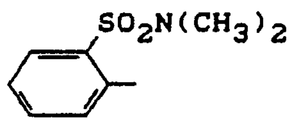 | $CH_3$ | $OCH_3$ | $OCH_3$ | CH | 149 |
| 121 | 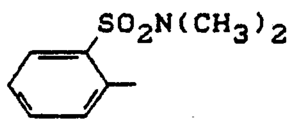 | $CH_3$ | H | $CH_3$ | CH | 160 |
| 122 | 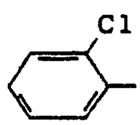 | $CH_3$ | H | $CH_3$ | CH | 152 |
| 123 | 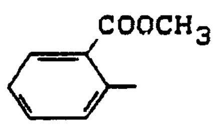 | $CH_3$ | $CH_3$ | $CH_3$ | CH | 143 |
| 124 | 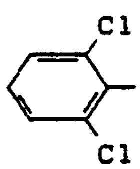 | $CH_3$ | H | $CH_3$ | CH | 161 |
| 125 | 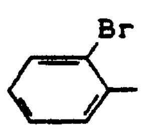 | $CH_3$ | H | $CH_3$ | CH | 142 |
| 126 | 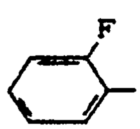 | $CH_3$ | H | $CH_3$ | CH | 118 |

Tabelle 1: Beispiele für die Verbindungen der Formel (I)

| Bsp.-Nr. | $R^1$ | $R^2$ | X | Y | Z | Schmelzpunkt (°C) |
|---|---|---|---|---|---|---|
| 127 | 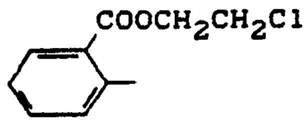 | $CH_3$ | H | $CH_3$ | CH | 95 |
| 128 | 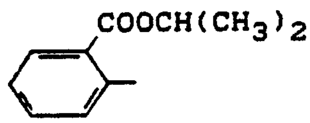 | $CH_3$ | H | $CH_3$ | CH | 90 |
| 129 | 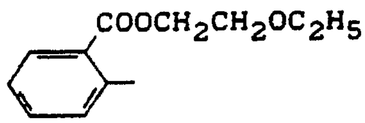 | $CH_3$ | H | $CH_3$ | CH | 100 |
| 130 | 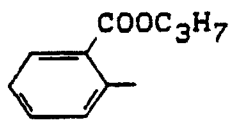 | $CH_3$ | H | $CH_3$ | CH | 100 |
| 131 | 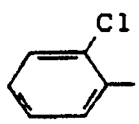 | $CH_3$ | $OCH_3$ | $OCH_3$ | CH | 168 |
| 132 | 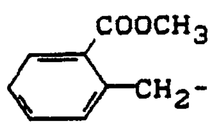 | $CH_3$ | $OCH_3$ | $OCH_3$ | CH | 142 |
| 133 | 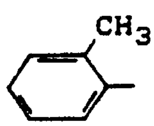 | $CH_3$ | $OCH_3$ | $OCH_3$ | N | 159 |
| 134 | 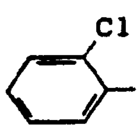 | $CH_3$ | $CH_3$ | $CH_3$ | CH | 157 |

Tabelle 1: Beispiele für die Verbindungen der Formel (I)

| Bsp.-Nr. | $R^1$ | $R^2$ | X | Y | Z | Schmelzpunkt (°C) |
|---|---|---|---|---|---|---|
| 135 | 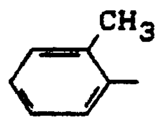 | $CH_3$ | $CH_3$ | $OCH_3$ | CH | 163 |
| 136 | 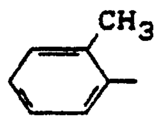 | $CH_3$ | $N(CH_3)_2$ | $CH_3$ | N | 157 |
| 137 | 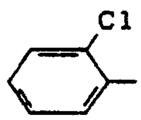 | $CH_3$ | $CH_3$ | $OCH_3$ | CH | 148 |
| 138 | 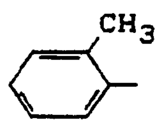 | $CH_3$ | 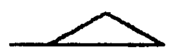 | 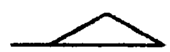 | N | 170 |
| 139 | 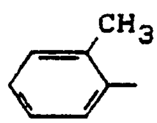 | $CH_3$ | $CH_3$ | $OC_2H_5$ | N | 104 |
| 140 | 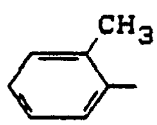 | $CH_3$ | $CH_3$ | $CH_3$ | N | 119 |
| 141 | 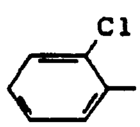 | $CH_3$ | $N(CH_3)_2$ | $CH_3$ | N | 175 |
| 142 | 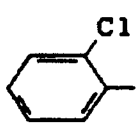 | $CH_3$ | $CH_3$ | $OCH_3$ | N | 136 |

Tabelle 1: Beispiele für die Verbindungen der Formel (I)

| Bsp.-Nr. | $R^1$ | $R^2$ | X | Y | Z | Schmelzpunkt (°C) |
|---|---|---|---|---|---|---|
| 143 | 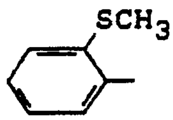 | $CH_3$ | $CH_3$ | $OCH_3$ | CH | 163 |
| 144 | 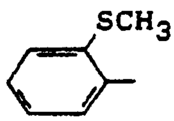 | $CH_3$ | $CH_3$ | $OCH_3$ | N | 167 |
| 145 | 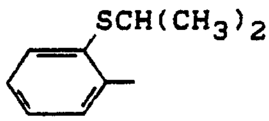 | $CH_3$ | $CH_3$ | $OCH_3$ | N | 163 |
| 146 | 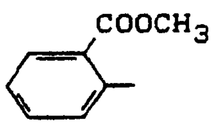 | $CH_3$ | $CH_3$ | $OCH_3$ | CH | 143 |
| 147 | 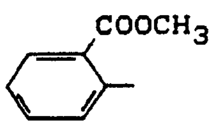 | $CH_3$ | $CH_3$ | $OCH_3$ | N | 160 |
| 148 | 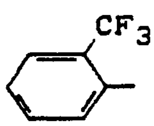 | $CH_3$ | $CH_3$ | $CH_3$ | N | 132 |
| 149 | 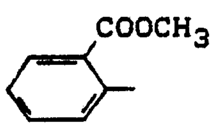 | $CH_3$ | $OCH_3$ | $OCH_3$ | N | 155 |
| 150 | 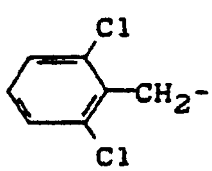 | $CH_3$ | $OCH_3$ | $OCH_3$ | CH | 190 |

<u>Tabelle 1:</u> Beispiele für die Verbindungen der Formel (I)

| Bsp.-Nr. | $R^1$ | $R^2$ | X | Y | Z | Schmelzpunkt (°C) |
|---|---|---|---|---|---|---|
| 151 | 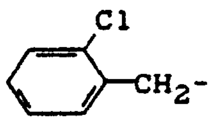 | $CH_3$ | $OCH_3$ | $OCH_3$ | CH | 144 |
| 152 | 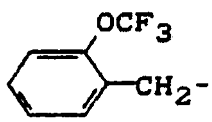 | $CH_3$ | $OCH_3$ | $OCH_3$ | CH | 173 |
| 153 | 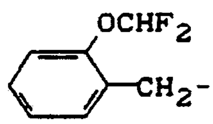 | $CH_3$ | $OCH_3$ | $OCH_3$ | CH | 139 |
| 154 | 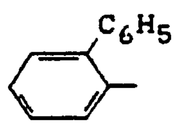 | $CH_3$ | $CH_3$ | $CH_3$ | CH | 129 |
| 155 | 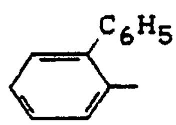 | $CH_3$ | $OCH_3$ | $OCH_3$ | CH | 130 |
| 156 | 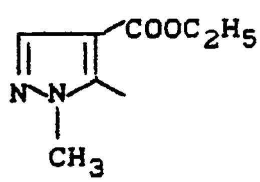 | $CH_3$ | H | $CH_3$ | CH | 118 |
| 157 | 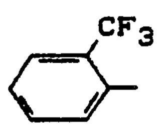 | $CH_3$ | H | $CH_3$ | CH | 126 |
| 158 | 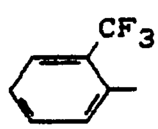 | $CH_3$ | $OCH_3$ | $OCH_3$ | CH | 155 |

Tabelle 1: Beispiele für die Verbindungen der Formel (I)

| Bsp.-Nr. | $R^1$ | $R^2$ | X | Y | Z | Schmelzpunkt (°C) |
|---|---|---|---|---|---|---|
| 159 | 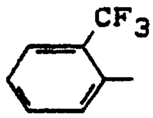 | $CH_3$ | $CH_3$ | $CH_3$ | CH | 141 |
| 160 | 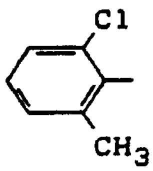 | $CH_3$ | H | $CH_3$ | CH | 126 |
| 161 | 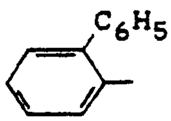 | $CH_3$ | $CH_3$ | $OCH_3$ | N | 168 |
| 162 | 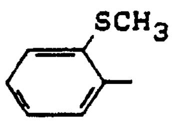 | $CH_3$ | $OCH_3$ | $OCH_3$ | CH | 186 |
| 163 | 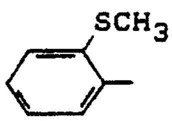 | $CH_3$ | $CH_3$ | $CH_3$ | CH | 135 |
| 164 | 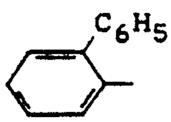 | $CH_3$ | $OCH_3$ | $OCH_3$ | N | 122 |
| 165 | 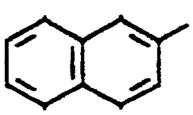 | $CH_3$ | $OCH_3$ | $OCH_3$ | N | 144 |
| 166 | 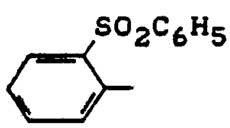 | $CH_3$ | $OCH_3$ | $OCH_3$ | N | 183 |

<u>Tabelle 1:</u> Beispiele für die Verbindungen der Formel (I)

| Bsp.-Nr. | $R^1$ | $R^2$ | X | Y | Z | Schmelzpunkt (°C) |
|---|---|---|---|---|---|---|
| 167 | 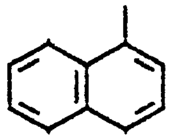 | $CH_3$ | $OCH_3$ | $OCH_3$ | N | 143 |
| 168 | 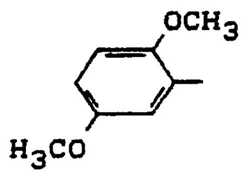 | $CH_3$ | $OCH_3$ | $OCH_3$ | N | 223 |
| 169 | 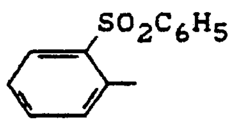 | $CH_3$ | $CH_3$ | $CH_3$ | CH | 212 |
| 170 | 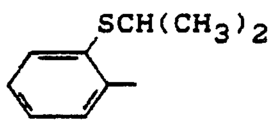 | $CH_3$ | $OCH_3$ | $OCH_3$ | CH | 108 |
| 171 | 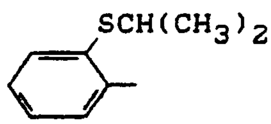 | $CH_3$ | $OCH_3$ | $OCH_3$ | N | |
| 172 | 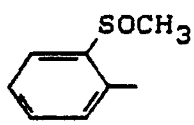 | $CH_3$ | $CH_3$ | $CH_3$ | CH | 180 |
| 173 | 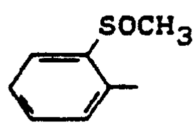 | $CH_3$ | $OCH_3$ | $OCH_3$ | CH | 117 |
| 174 | 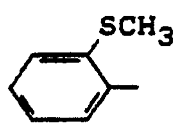 | $CH_3$ | $OCH_3$ | $OCH_3$ | N | 162 |

<u>Tabelle 1:</u> Beispiele für die Verbindungen der Formel (I)

| Bsp.-Nr. | $R^1$ | $R^2$ | X | Y | Z | Schmelzpunkt (°C) |
|---|---|---|---|---|---|---|
| 175 | 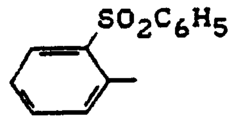 | $CH_3$ | $OCH_3$ | $OCH_3$ | CH | 204 |
| 176 | 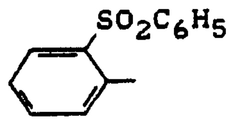 | $CH_3$ | $CH_3$ | $OCH_3$ | N | 179 |
| 177 | 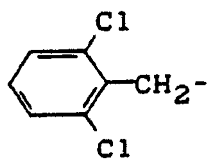 | $CH_3$ | $C_2H_5$ | $OCH_3$ | N | 164 |
| 178 | 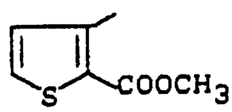 | $CH_3$ | $CH_3$ | $OC_2H_5$ | N | 115 |
| 179 | 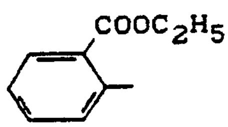 | $CH_3$ | $OCH_3$ | $OCH_3$ | N | 160 |
| 180 | 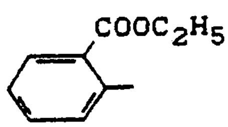 | $CH_3$ | $CH_3$ | $OCH_3$ | N | 145 |
| 181 | 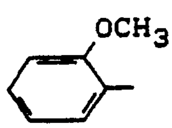 | $CH_3$ | $CH_3$ | $CH_3$ | CH | 134 |
| 182 | 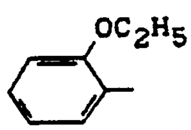 | $CH_3$ | $CH_3$ | $CH_3$ | CH | 163 |

<u>Tabelle 1:</u> Beispiele für die Verbindungen der Formel (I)

| Bsp.-Nr. | $R^1$ | $R^2$ | X | Y | Z | Schmelzpunkt (°C) |
|---|---|---|---|---|---|---|
| 183 | 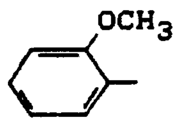 | $CH_3$ | $OCH_3$ | $OCH_3$ | CH | 205 |
| 184 | 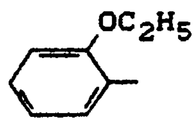 | $CH_3$ | $OCH_3$ | $OCH_3$ | CH | 198 |
| 185 | 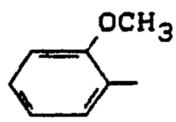 | $CH_3$ | H | $CH_3$ | CH | 119 |
| 186 | 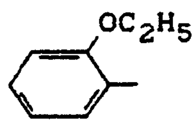 | $CH_3$ | H | $CH_3$ | CH | 150 |
| 187 | 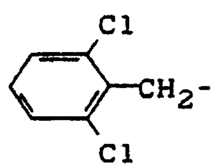 | $CH_3$ | H | $CH_3$ | CH | 128 |
| 188 | 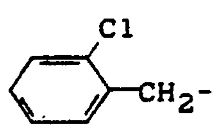 | $CH_3$ | H | $CH_3$ | CH | 139 |
| 189 | 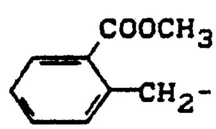 | $CH_3$ | H | $CH_3$ | CH | 102 |
| 190 | 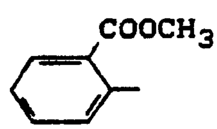 | $CH_3$ | H | $OCH_3$ | $CCH_3$ | 156 |

<u>Tabelle 1:</u> Beispiele für die Verbindungen der Formel (I)

| Bsp.-Nr. | $R^1$ | $R^2$ | X | Y | Z | Schmelzpunkt (°C) |
|---|---|---|---|---|---|---|
| 191 | 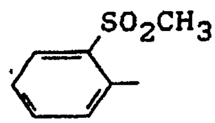 | $CH_3$ | $SCH_3$ | $CH_3$ | N | 194 |
| 192 | 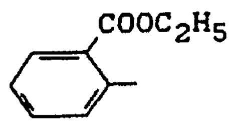 | $CH_3$ | $SCH_3$ | $CH_3$ | N | 112 |
| 193 | 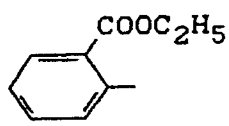 | $CH_3$ | $OC_2H_5$ | $CH_3$ | N | 109 |
| 194 | 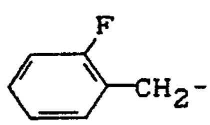 | $CH_3$ | $OCH_3$ | $CH_3$ | N | 155 |
| 195 | 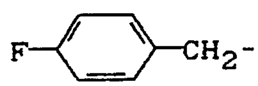 | $CH_3$ | $OCH_3$ | $CH_3$ | N | 172 |
| 196 | 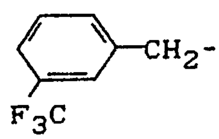 | $CH_3$ | $OCH_3$ | $CH_3$ | N | 184 |
| 197 | 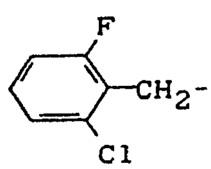 | $CH_3$ | $OCH_3$ | $CH_3$ | N | 202 |
| 198 | 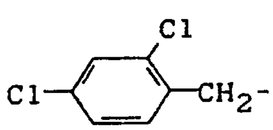 | $CH_3$ | $OCH_3$ | $CH_3$ | N | 144 |

Tabelle 1: Beispiele für die Verbindungen der Formel (I)

| Bsp.-Nr. | $R^1$ | $R^2$ | X | Y | Z | Schmelzpunkt (°C) |
|---|---|---|---|---|---|---|
| 199 | 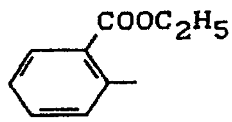 | $CH_3$ | $OCH_3$ | $C_2H_5$ | N | 112 |
| 200 | 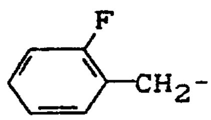 | $CH_3$ | $OCH_3$ | $OCH_3$ | N | 155 |
| 201 | 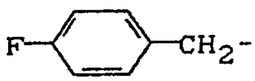 | $CH_3$ | $OCH_3$ | $OCH_3$ | N | 185 |
| 202 | 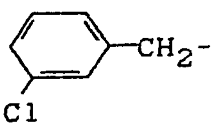 | $CH_3$ | $OCH_3$ | $OCH_3$ | N | 179 |
| 203 | 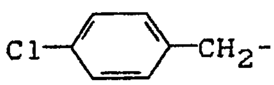 | $CH_3$ | $OCH_3$ | $OCH_3$ | N | 178 |
| 204 | 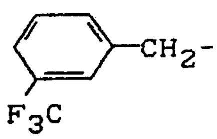 | $CH_3$ | $OCH_3$ | $OCH_3$ | N | 176 |
| 205 | 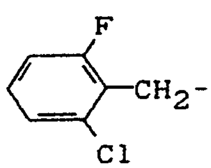 | $CH_3$ | $OCH_3$ | $OCH_3$ | N | 190 |
| 206 | 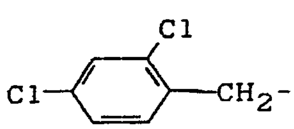 | $CH_3$ | $OCH_3$ | $OCH_3$ | N | 163 |

**Tabelle 1:** Beispiele für die Verbindungen der Formel (I)

| Bsp.-Nr. | $R^1$ | $R^2$ | X | Y | Z | Schmelzpunkt (°C) |
|---|---|---|---|---|---|---|
| 207 | 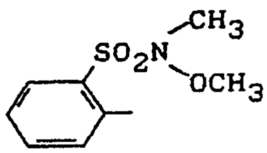 | $CH_3$ | $OCH_3$ | $C_2H_5$ | N | 125 |
| 208 | 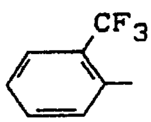 | $CH_3$ | $OCH_3$ | $C_2H_5$ | N | 150 |
| 209 | 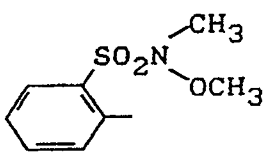 | $CH_3$ | $OCH_3$ | $CH_3$ | N | 154 |
| 210 | 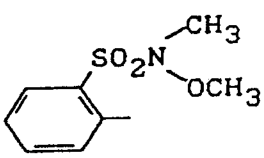 | $CH_3$ | $OCH_3$ | $OCH_3$ | N | 140 |
| 211 | 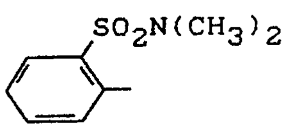 | $CH_3$ | $OC_2H_5$ | $CH_3$ | N | 115 |
| 212 | 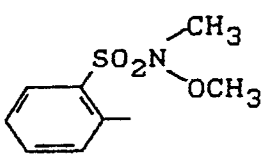 | $CH_3$ | $OC_2H_5$ | $CH_3$ | N | 118 |
| 213 | 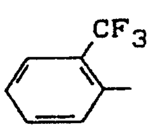 | $CH_3$ | $OC_2H_5$ | $CH_3$ | N | 136 |

Tabelle 1: Beispiele für die Verbindungen der Formel (I)

| Bsp.-Nr. | $R^1$ | $R^2$ | X | Y | Z | Schmelzpunkt (°C) |
|---|---|---|---|---|---|---|
| 214 | 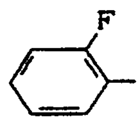 | $CH_3$ | $OC_2H_5$ | $CH_3$ | N | 98 |
| 215 | 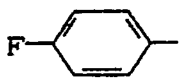 | $CH_3$ | $OCH_3$ | $OCH_3$ | N | 188 |
| 216 | 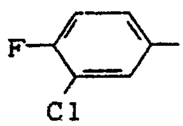 | $CH_3$ | $OCH_3$ | $OCH_3$ | N | 164 |
| 217 | 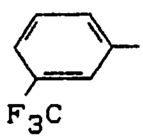 | $CH_3$ | $OCH_3$ | $OCH_3$ | N | 142 |
| 218 | 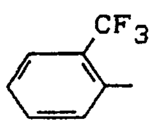 | $CH_3$ | $CH_3$ | $CH_3$ | N | 138 |
| 219 | 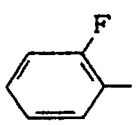 | $CH_3$ | $SCH_3$ | $CH_3$ | N | 143 |
| 220 | 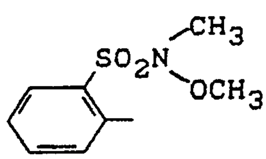 | $CH_3$ | $SCH_3$ | $CH_3$ | N | 137 |
| 221 | 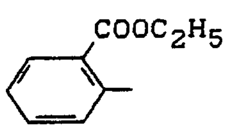 | $CH_3$ | $CH_3$ | $CH_3$ | N | 134 |

<u>Tabelle 1:</u> Beispiele für die Verbindungen der Formel (I)

| Bsp.-Nr. | $R^1$ | $R^2$ | X | Y | Z | Schmelzpunkt (°C) |
|---|---|---|---|---|---|---|
| 222 | 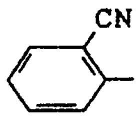 | $CH_3$ | $OCH_3$ | $CH_3$ | N | 165 |
| 223 | 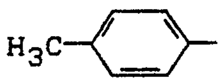 | $CH_3$ | $OCH_3$ | $CH_3$ | N | 215 |
| 224 | 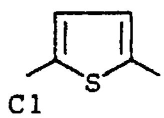 | $CH_3$ | $OCH_3$ | $CH_3$ | N | 162 |
| 225 | 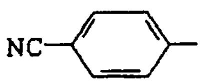 | $CH_3$ | $OCH_3$ | $OCH_3$ | N | 218 |
| 226 | 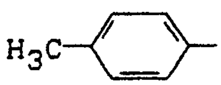 | $CH_3$ | $OCH_3$ | $OCH_3$ | N | 205 |
| 227 | 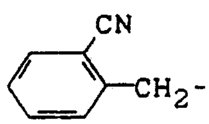 | $CH_3$ | $OCH_3$ | $OCH_3$ | N | 193 |
| 228 | 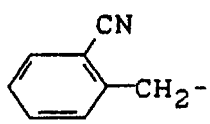 | $CH_3$ | $OCH_3$ | $CH_3$ | N | 242 |
| 229 | 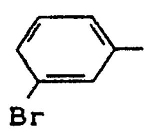 | $CH_3$ | $OCH_3$ | $OCH_3$ | N | 147 |
| 230 | 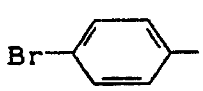 | $CH_3$ | $OCH_3$ | $OCH_3$ | N | 201 |

Tabelle 1: Beispiele für die Verbindungen der Formel (I)

| Bsp.-Nr. | $R^1$ | $R^2$ | X | Y | Z | Schmelzpunkt (°C) |
|---|---|---|---|---|---|---|
| 231 | 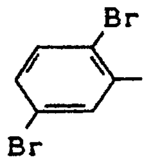 | $CH_3$ | $OCH_3$ | $OCH_3$ | N | 231 |
| 232 | 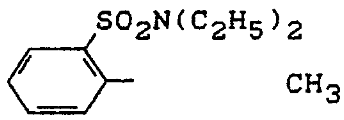 | $CH_3$ | $OCH_3$ | $OCH_3$ | N | 147 |
| 233 | 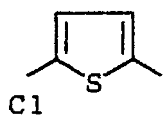 | $CH_3$ | $OCH_3$ | $OCH_3$ | N | 162 |
| 234 | 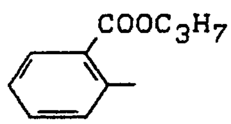 | $CH_3$ | $OCH_3$ | $CH_3$ | N | 130 |
| 235 | 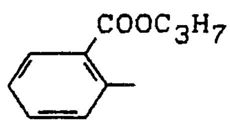 | $CH_3$ | $OC_2H_5$ | $CH_3$ | N | 109 |
| 236 | 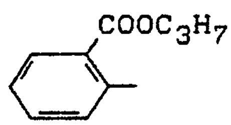 | $CH_3$ | $OCH_3$ | $C_2H_5$ | N | 116 |
| 237 | 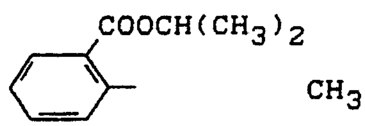 | $CH_3$ | $OCH_3$ | $CH_3$ | N | 133 |
| 238 | 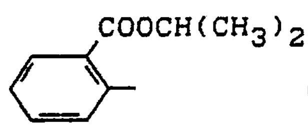 | $CH_3$ | $OC_2H_5$ | $CH_3$ | N | 109 |

Tabelle 1: Beispiele für die Verbindungen der Formel (I)

| Bsp.-Nr. | $R^1$ | $R^2$ | X | Y | Z | Schmelzpunkt (°C) |
|---|---|---|---|---|---|---|
| 239 | 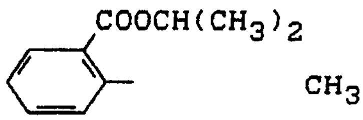 | $CH_3$ | $OCH_3$ | $C_2H_5$ | N | 143 |
| 240 | 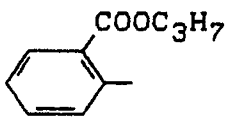 | $CH_3$ | $OCH_3$ | $OCH_3$ | N | 146 |
| 241 | 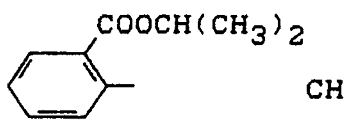 | $CH_3$ | $OCH_3$ | $OCH_3$ | N | 137 |
| 242 | 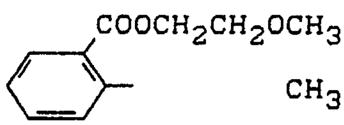 | $CH_3$ | $OCH_3$ | $OCH_3$ | N | 124 |
| 243 | 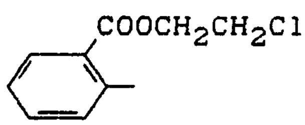 | $CH_3$ | $OCH_3$ | $OCH_3$ | N | 155 |
| 244 | 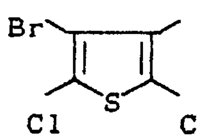 | $CH_3$ | $OCH_3$ | $OCH_3$ | N | 150 |
| 245 | 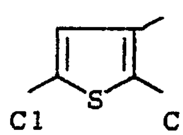 | $CH_3$ | $OCH_3$ | $OCH_3$ | N | 166 |
| 246 | 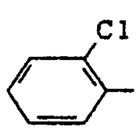 | $CH_3$ | $OCH_3$ | $CH_3$ | N | 149 |

<u>Tabelle 1:</u> Beispiele für die Verbindungen der Formel (I)

| Bsp.-Nr. | $R^1$ | $R^2$ | X | Y | Z | Schmelzpunkt (°C) |
|---|---|---|---|---|---|---|
| 247 | 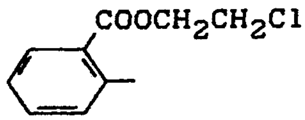 | $CH_3$ | $OCH_3$ | $CH_3$ | N | 127 |
| 248 | 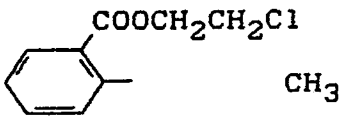 | $CH_3$ | $OC_2H_5$ | $CH_3$ | N | 135 |
| 249 | 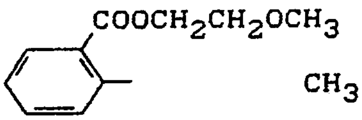 | $CH_3$ | $OC_2H_5$ | $CH_3$ | N | 102 |
| 250 | 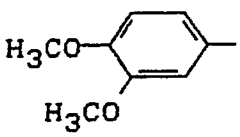 | $CH_3$ | $OCH_3$ | $CH_3$ | N | 195 |
| 251 | 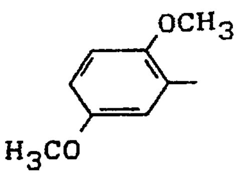 | $CH_3$ | $OCH_3$ | $CH_3$ | N | 165 |
| 252 | 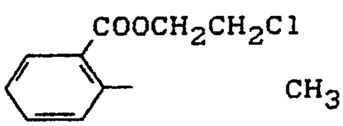 | $CH_3$ | $OCH_3$ | $C_2H_5$ | N | 129 |
| 253 | 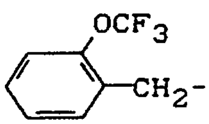 | $CH_3$ | $CH_3$ | $CH_3$ | CH | 151 |
| 254 | 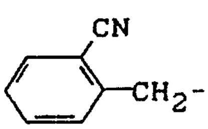 | $CH_3$ | $CH_3$ | $CH_3$ | CH | 179 |

Tabelle 1: Beispiele für die Verbindungen der Formel (I)

| Bsp.-Nr. | $R^1$ | $R^2$ | X | Y | Z | Schmelzpunkt (° C) |
|---|---|---|---|---|---|---|
| 255 | 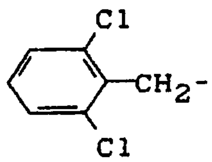 | $CH_3$ | $CH_3$ | $CH_3$ | CH | 209 |
| 256 | 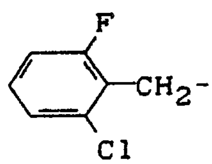 | $CH_3$ | $CH_3$ | $CH_3$ | CH | 201 |
| 257 | 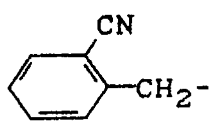 | $CH_3$ | $OCH_3$ | $CH_3$ | CH | 167 |
| 258 | 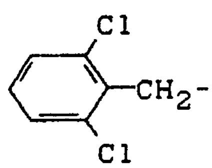 | $CH_3$ | $OCH_3$ | $CH_3$ | CH | 201 |
| 259 | 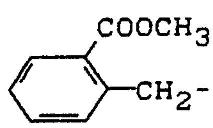 | $CH_3$ | $OCH_3$ | $CH_3$ | CH | 142 |
| 260 | 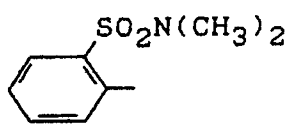 | $CH_3$ | $OCH_3$ | $CH_3$ | CH | 160 |
| 261 | 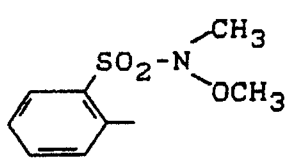 | $CH_3$ | $OCH_3$ | $CH_3$ | CH | 163 |

Tabelle 1: Beispiele für die Verbindungen der Formel (I)

| Bsp.-Nr. | $R^1$ | $R^2$ | X | Y | Z | Schmelzpunkt (°C) |
|---|---|---|---|---|---|---|
| 262 | 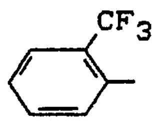 | $CH_3$ | $OCH_3$ | $CH_3$ | CH | 149 |
| 263 | 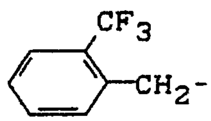 | $CH_3$ | $OCH_3$ | $CH_3$ | CH | 147 |
| 264 | 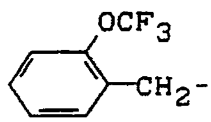 | $CH_3$ | $OCH_3$ | $CH_3$ | CH | 159 |
| 265 | 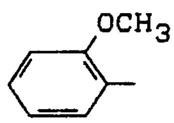 | $CH_3$ | $OCH_3$ | $CH_3$ | CH | 152 |
| 266 | 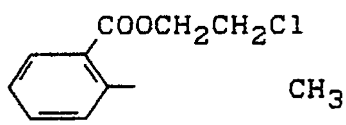 | $CH_3$ | $OCH_3$ | H | $C-CH_3$ | 143 |
| 267 | 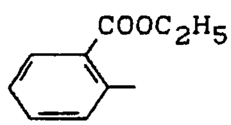 | $CH_3$ | $OCH_3$ | H | $C-CH_3$ | 132 |
| 268 | 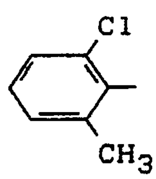 | $CH_3$ | $OCH_3$ | H | $C-CH_3$ | 152 |
| 269 | 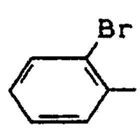 | $CH_3$ | $OCH_3$ | H | $C-CH_3$ | 171 |

**Tabelle 1:** Beispiele für die Verbindungen der Formel (I)

| Bsp.-Nr. | $R^1$ | $R^2$ | X | Y | Z | Schmelzpunkt (°C) |
|---|---|---|---|---|---|---|
| 270 | 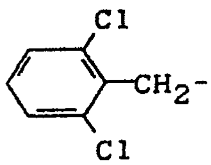 | $CH_3$ | $OCH_3$ | H | $C-CH_3$ | 198 |
| 271 | 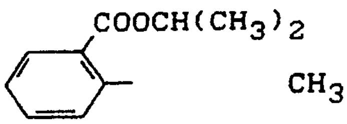 | $CH_3$ | $OCH_3$ | $OCH_3$ | N | 139 |
| 272 | 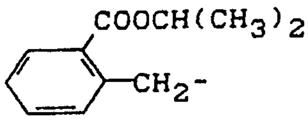 | $CH_3$ | $OCH_3$ | $OCH_3$ | CH | 155 |
| 273 | 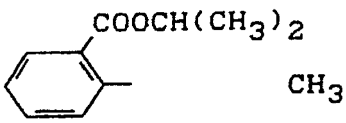 | $CH_3$ | $CH_3$ | $CH_3$ | CH | 112 |
| 274 | 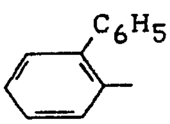 | $CH_3$ | $OCH_3$ | $CH_3$ | CH | 136 |
| 275 | 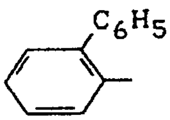 | $CH_3$ | $CH_3$ | $CH_3$ | N | 154 |
| 276 | 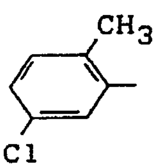 | $CH_3$ | $OCH_3$ | $OCH_3$ | CH | 178 |
| 277 | 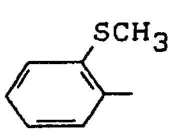 | $CH_3$ | $OC_2H_5$ | $OC_2H_5$ | CH | 122 |

Von den in Tabelle 1 aufgeführten Verbindungen sind insbesondere die als Beispiele Nr. 13, 15, 16, 17, 18, 46, 47, 48, 49, 50, 54, 60, 61, 63, 64, 73, 148 und 149 genannten Verbindungen für die Verwendung als selektive Herbizide geeignet.

Die Sulfonylisothioharnstoffe der Formel (I) sind bekannt und/oder können nach an sich bekannten Verfahren hergestellt werden (vgl. EP-A 5986; EP-A 23141; EP-A 30142; EP-A 173311; JP-A 62-148482, zit. in Chem. Abstracts 107 (1987), 236729y; DE-OS 3618004/LeA 24460).

Noch nicht aus der Literatur bekannt sind beispielsweise die in Tabelle 1 als Beispiele Nr. 13, 16, 48, 49, 50, 60, 61 und 64 aufgeführten Verbindungen, welche im Folgenden namentlich genannt sind:

N′-(4,6-Dimethoxy-s-triazin-2-yl)-N″-(2-trifluormethylphenylsulfonyl)-S-methyl-isothioharnstoff, N′-(4-

Methoxy-6-methyl-s-triazin-2-yl)-N''-(2-chlor-6-methyl-phenylsulfonyl)-S-methyl-isothioharnstoff, N'-(4-Methoxy-6-methyl-s-triazin-2-yl)-N''-(2,5-dichlor-3-thienylsulfonyl)-S-methyl-isothioharnstoff, N'-(4-Methoxy-6-methyl-s-triazin-2-yl)-N''-(2-fluor-phenylsulfonyl)-S-methyl-isothioharnstoff, N'-(4-Methoxy-6-methyl-s-triazin-2-yl)-N''-(2-brom-phenylsulfonyl)-S-methyl-isothioharnstoff, N'-(4,6-Dimethoxy-s-triazin-2-yl)-N''-(2-chlor-6-methyl-phenylsulfonyl)-S-methyl-isothioharnstoff, N'-(4,6-Dimethoxy-s-triazin-2-yl)-N''-(2,6-dichlor-phenylsulfonyl)-S-methyl-isothioharnstoff und N'-(4-Methyl-6-methylthio-s-triazin-2-yl)-N''-(2-methoxycarbonyl-phenylsulfonyl)-S-methyl-isothioharnstoff.

Diese neuen Verbindungen der Formel (I) können analog zu bekannten Verfahren hergestellt werden (vgl. auch die Herstellungsbeispiele).

Neu sind weiter die eine Auswahl aus den Verbindungen der Formel (I) darstellenden Sulfonylisothioharnstoffe der allgemeinen Formel (Ia)

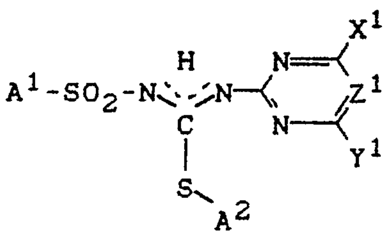

(Ia)

in welcher

$A^1$ für Benzyl, welches durch $C_1$-$C_4$-Halogenalkyl, $C_1$-$C_4$-Halogenalkoxy oder $C_1$-$C_4$-Alkoxycarbonyl substituiert ist, oder für Pyridylmethyl, welches durch Halogen, $C_1$-$C_4$-Alkyl, $C_1$-$C_4$-Halogenalkyl, $C_1$-$C_4$-Alkoxy oder $C_1$-$C_4$-Halogenalkoxy substituiert ist, steht,

$A^2$ für $C_1$-$C_4$-Alkyl (welches gegebenenfalls durch Halogen, Cyano, $C_1$-$C_4$-Alkoxy, Carboxy, $C_1$-$C_4$-Alkoxycarbonyl, Aminocarbonyl, $C_1$-$C_4$-Alkylamino-carbonyl oder Di-($C_1$-$C_4$-alkyl)-amino-carbonyl sustituiert ist), für Benzyl oder für Phenylethyl steht (welche jeweils gegebenenfalls durch Halogen, $C_1$-$C_4$-Alkyl, $C_1$-$C_4$-Alkoxy oder $C_1$-$C_4$-Alkoxy-carbonyl substituiert sind),

$X^1$ für Wasserstoff, Halogen, $C_1$-$C_4$-Alkyl, $C_1$-$C_4$-Halogenalkyl, Cyclopropyl, $C_1$-$C_4$-Alkoxy, $C_1$-$C_4$-Alkylthio, $C_1$-$C_4$-Alkylamino oder Di-($C_1$-$C_4$-alkyl)-amino steht,

$Y^1$ für $C_1$-$C_4$-Alkyl, $C_1$-$C_4$-Halogenalkyl, Cyclopropyl oder $C_1$-$C_4$-Alkoxy steht und

$Z^1$ für Stickstoff oder eine CH-Gruppierung steht.

Bevorzugt sind die neuen Verbindungen der Formel (Ia), in welcher

$A^1$ für Benzyl, welches in ortho-Position durch Difluormethoxy, Trifluormethoxy oder Methoxycarbonyl substituiert ist, oder für Pyridylmethyl, welches durch Fluor, Chlor, Brom, Methyl, Trifluormethyl oder Methoxy substituiert ist, steht,

$A^2$ für Methyl, Ethyl, Cyanomethyl, Carboxymethyl, Methoxycarbonylmethyl, Ethoxycarbonylmethyl oder Benzyl steht,

$X^1$ für Methyl, Ethyl, Trifluormethyl, Cyclopropyl, Methoxy oder Ethoxy steht,

$Y^1$ für Methyl, Ethyl, Trifluormethyl, Cyclopropyl, Methoxy oder Ethoxy steht und

$Z^1$ für Stickstoff oder eine CH-Gruppierung steht.

Die neuen Verbindungen der Formel (Ia) können analog zu bekannten Verfahren hergestellt werden (vgl. auch die Herstellungsbeispiele).

Man erhält die neuen Sulfonylisothioharnstoffe der allgemeinen Formel (Ia) beispielsweise, wenn man Sulfonsäurechloride der allgemeinen Formel (II)

$A^1$-$SO_2$-Cl (II)

in welcher

$A^1$ die oben angegebene Bedeutung hat,

mit Isothioharnstoffen der allgemeinen Formel (III)

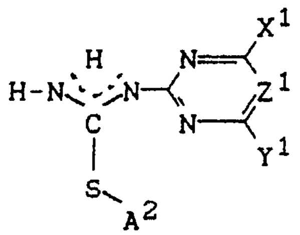

(III)

in welcher
$A^2$, $X^1$, $Y^1$ und $Z^1$ die oben angegebene Bedeutung haben,
gegebenenfalls in Gegenwart von Säureakzeptoren, wie z.B. Pyridin oder 1,4-Diazabicyclo-[2,2,2]-octan (DABCO), und gegebenenfalls in Gegenwart von Verdünnungsmitteln, wie z.B. Methylenchlorid oder Chloroform, bei Temperaturen zwischen -20° C und +30° C umsetzt und nach üblichen Methoden aufarbeitet (vgl. Herstellungsbeispiele).

Bei den Ausgangsstoffen der Formeln (II) und (III) haben $A^1$ bzw. $A^2$, $X^1$, $Y^1$ und $Z^1$ vorzugsweise die gleichen Bedeutungen, wie sie oben im Rahmen der Beschreibung der neuen Verbindungen der Formel (Ia) als bevorzugt angegeben sind.

Die Ausgangsstoffe der Formel (II) sind bereits bekannt und/oder können nach an sich bekannten Verfahren hergestellt werden (vgl. US-P 4806147).

Die Ausgangsstoffe der Formel (III) sind ebenfalls bekannt und/oder können nach an sich bekannten Verfahren hergestellt werden (vgl. EP-A 117014).

Die erfindungsgemäß zu verwendenden Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Cheno podium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Zierge hölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die Verbindungen der Formel (I) eignen sich insbesondere zur selektiven Bekämpfung von dikotylen Unkräutern in monokotylen Kulturen, wie z.B. Weizen, im Vorauflauf-und im Nachauflauf-Verfahren.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide, Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N′-dimethyl-harnstoff (METABENZTHIAZURON) zur Unkrautbekämpfung in Getreide; 4-Amino-3-methyl-6-phenyl-1,2,4-triazin-5-(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, in Frage; ferner auch 2,4-Dichlorphenoxyessigsäure (2,4-D); 4-(2,4-Dichlorphenoxy)-buttersäure (2,4-DB); 2,4-Dichlorphenoxypropionsäure (2,4-DP); 2-Chlor-4-ethylamino-6-isopropyl amino-1,3,5-triazin (ATRAZIN); 3-Isopropyl-2,1,3-benzothiadiazin-4-on-2,2-dioxid (BENTAZON); Methyl-5-(2,4-dichlorphenoxy)-2-nitrobenzoat (BIFENOX); 3,5-Dibrom-4-hydroxy-benzonitril (BROMOXYNIL); 2-Chlor-N-{[(4-methoxy-6-methyl-1,3,5-triazin-2-yl)-amino]-carbonyl}-benzolsulfonamid (CHLORSULFURON); N,N-Dimethyl-N′-(3-chlor-4-methylphenyl)-harnstoff (CHLORTOLURON); 2-Chlor-4-ethylamino-6-(3-cyanopropylamino)-1,3,5-triazin (CYANAZIN); 2-[4-(2,4-Dichlorphenoxy)-phenoxy]-propionsäure, deren Methyl-oder deren Ethylester (DICLOFOP); 4-Amino-6-t-butyl-3-ethylthio-1,2,4-triazin-5(4H)-on (ETHIOZIN); 2-{4-[(6-Chlor-2-benzoxazolyl)-oxy]-phenoxy}-propansäure, deren Methyl- oder deren Ethylester (FENOXAPROP); [(4-Amino-3,5-dichlor-6-fluor-2-pyridinyl)-oxy]-essigsäure bzw. deren 1-Methylheptylester (FLUROXYPYR); Methyl-2-[4,5-dihydro-4-methyl-4-(1-methylethyl)-5-oxo-1H-imidazol-2-yl]-4(5)-methylbenzoat (IMAZAMETHABENZ); 3,5-Diiod-4-hydroxybenzonitril (IOXYNIL); N,N-Dimethyl-N′-(4-isopropylphenyl)-harnstoff (ISOPROTURON); (2-Methyl-4-chlorphenoxy)-essigsäure (MCPA); (4-Chlor-2-methylphenoxy)-propionsäure (MCPP); N-Methyl-2-(1,3-benzthiazol-2-yloxy)-acetanilid (MEFENACET); 2-{[[((4-Methoxy-6-methyl-1,3,5-triazin-2-yl)-amino)-carbonyl]-amino]-sulfonyl}-benzoesäure oder deren Methylester (METSULFURON); N-(1-Ethylpropyl)-3,4-dimethyl-2,6-dinitroanilin (PENDIMETHALIN); O-(6-Chlor-3-phenyl-pyridazin-4-yl)-S-octylthiocarbonat (PYRIDATE); 4-Ethylamino-2-t-butylamino-6-methylthio-s-triazin (TERBUTRYNE); 3-[[[[(4-Methoxy-6-methyl-1,3,5-triazin-2-yl)-amino]-carbonyl]-amino]-sulfonyl]-thiophen-2-carbonsäure-methylester (THIAMETURON); N,N-Diisopropyl-S-(2,3,3-trichlorallyl)-thiolcarbamat (TRIALLATE). Einige Mischungen zeigen überraschender weise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

Anwendungsbeispiele:

Die Strukturformeln der für die Anwendungsbeispiele herangezogenen Verbindungen der Formel (I) sind nachstehend (mit den Beispiel-Nummern aus Tabelle 1) einzeln aufgeführt.

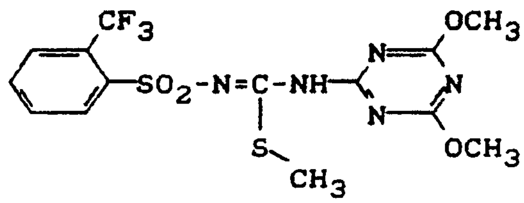

(13)

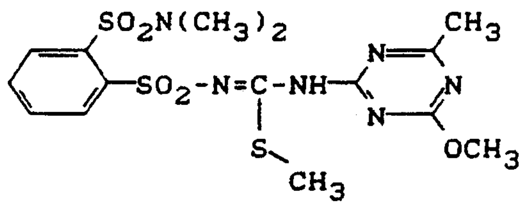

(15)

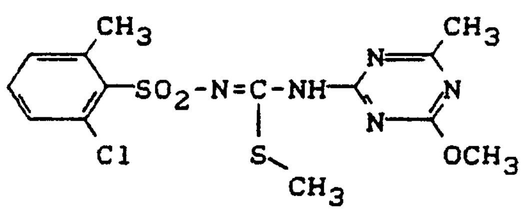

(16)

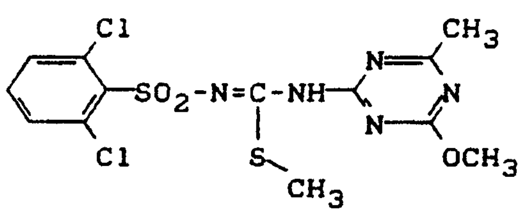

(17)

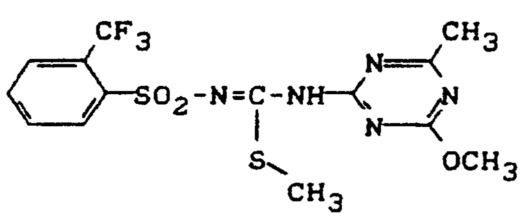

(18)

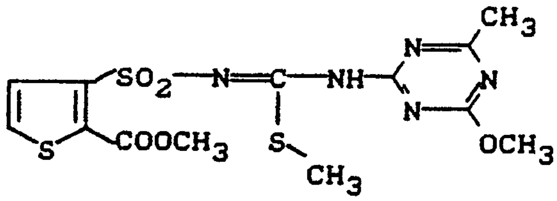

(46)

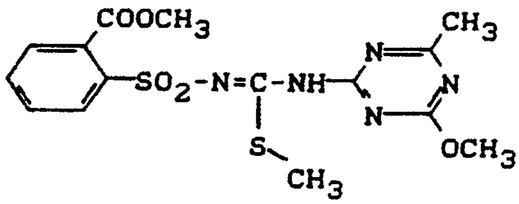

(47)

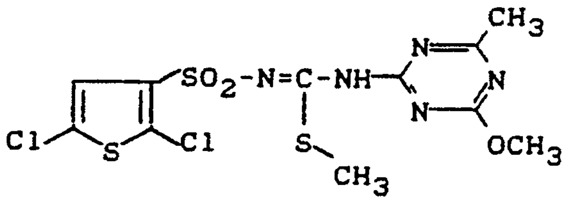

(48)

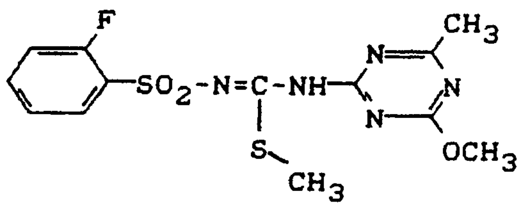

(49)

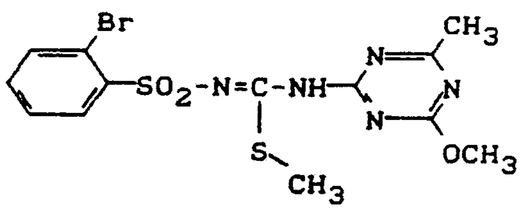

(50)

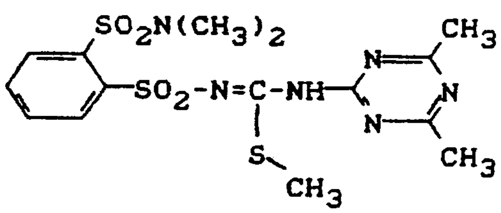

(54)

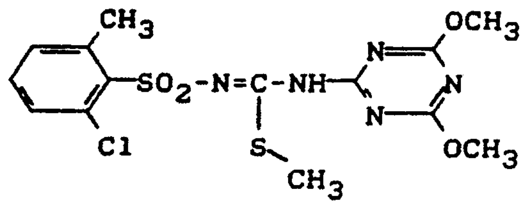

(60)

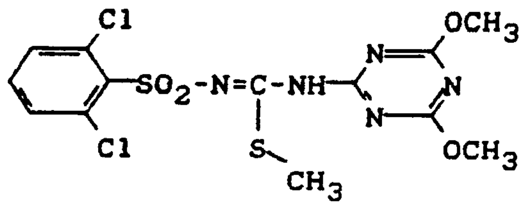

(61)

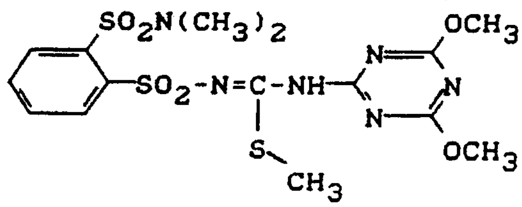

(63)

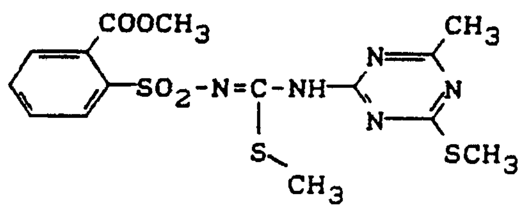

(64)

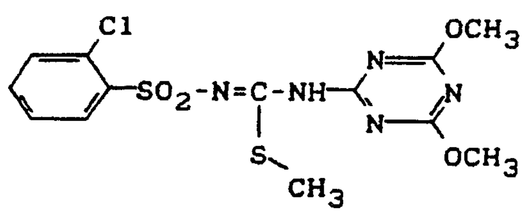

(73)

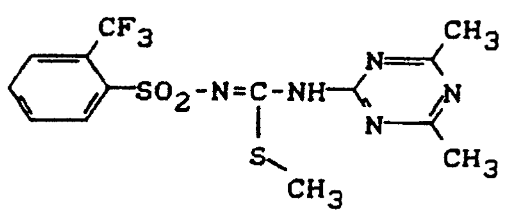

(148)

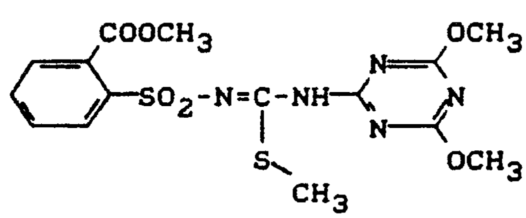

(149)

Beispiel A

Pre-emergence-Test

Lösungsmittel: 5 Gewichtsteile Aceton

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)

100 % = totale Vernichtung

In diesem Test zeigen beispielsweise die Verbindungen gemäß den Beispielen (13), (15), (16), (17), (18), (47), (49), (50), (60), (61), (63) und (148) bei sehr guter Verträglichkeit gegenüber Weizen sehr starke Wirkung gegen Unkräuter, wie z.B. Chenopodium, Galinsoga, Matricaria und Stellaria.

**Tabelle A:** Pre-emergence-Test/Gewächshaus

| Wirkstoff (Bsp.-Nr.) | Aufwandmenge (g/ha) | Weizen | Chenopodium | Galinsoga | Matricaria | Stellaria |
|---|---|---|---|---|---|---|
| 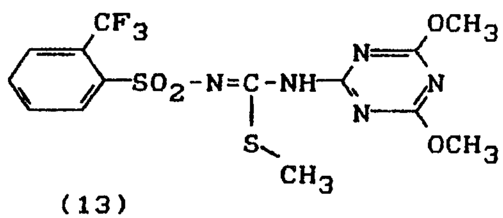 (13) | 125 | 0 | 70 | 80 | 95 | 90 |
| 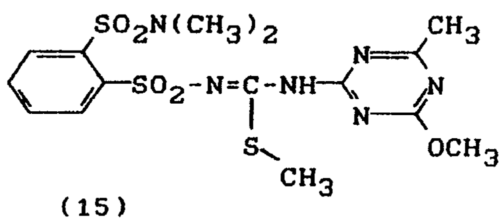 (15) | 125 | 0 | 100 | 95 | 95 | 95 |
| 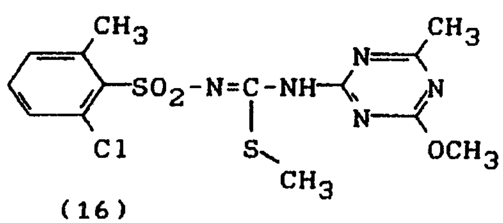 (16) | 125 | 10 | 100 | 95 | 95 | 95 |
| 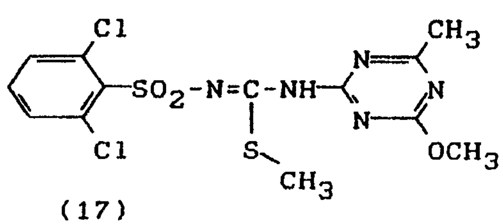 (17) | 125 | 0 | 90 | 90 | 90 | 95 |

Tabelle A:

| Wirkstoff (Bsp.-Nr.) | Aufwandmenge (g/ha) | Weizen | Chenopodium | Galinsoga | Matricaria | Stellaria |
|---|---|---|---|---|---|---|
| 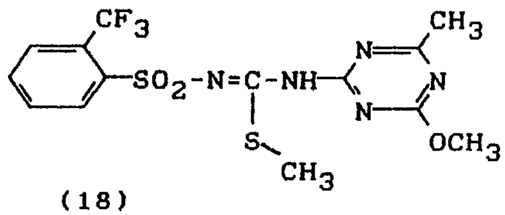 (18) | 125 | 0 | 100 | 95 | 95 | 95 |
| 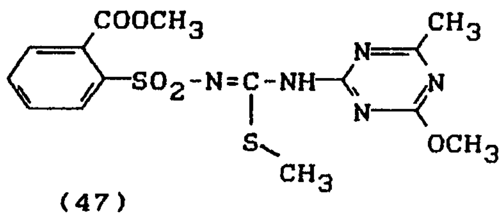 (47) | 125 | 0 | 100 | 95 | 100 | 95 |
| 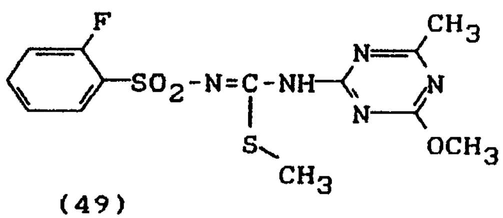 (49) | 125 | 0 | 100 | 30 | 70 | 90 |
| 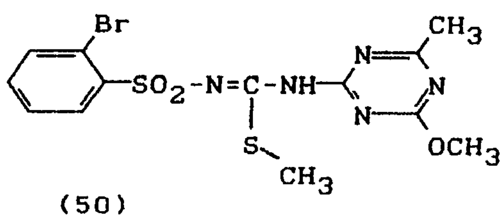 (50) | 125 | 0 | 95 | 90 | 95 | 95 |

**Tabelle A:**

| Wirkstoff (Bsp.-Nr.) | Aufwandmenge (g/ha) | Weizen | Chenopodium | Galinsoga | Matricaria | Stellaria |
|---|---|---|---|---|---|---|
| 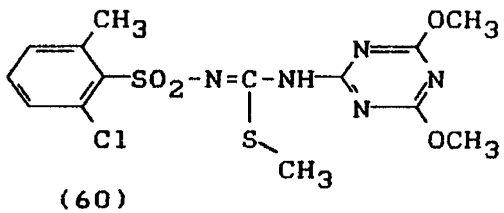 (60) | 125 | 20 | 80 | 80 | 90 | 80 |
| 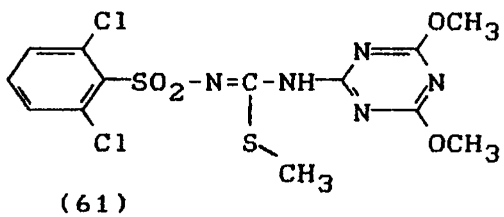 (61) | 125 | 0 | 90 | 90 | 100 | 90 |
| 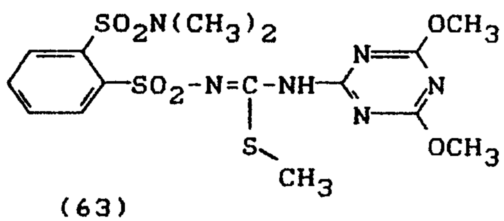 (63) | 125 | 0 | 60 | 60 | 90 | 90 |
| 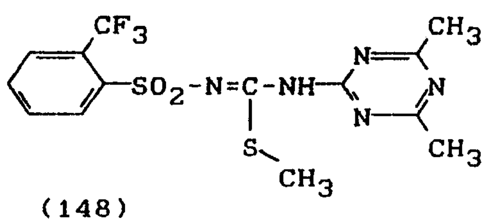 (148) | 125 | 0 | 90 | 90 | 90 | 80 |

Beispiel B

Post-emergence-Test

Lösungsmittel: 5 Gewichtsteile Aceton

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben, so, daß die jeweils gewünschten Wirkstoffmengen pro Flächeneinheit ausgebracht werden. Die Konzentration der Spritzbrühe wird so gewählt, daß in 1000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)

100 % = totale Vernichtung

In diesem Test zeigen beispielsweise die Verbindungen gemäß den Beispielen (13), (15), (16), (17), (18), (46), (47), (48), (49), (50), (54), (60), (61), (63), (64), (73), (148) und (149) bei sehr guter Verträglichkeit gegenüber Weizen sehr starke Wirkung gegen Unkräuter, wie z.B. Galinsoga, Matricaria, Sinapis und Stellaria.

**Tabelle B:** Post-emergence-Test/Gewächshaus

| Wirkstoff (Bsp.-Nr.) | Aufwandmenge (g/ha) | Weizen | Galinsoga | Matricaria | Sinapis | Stellaria |
|---|---|---|---|---|---|---|
| 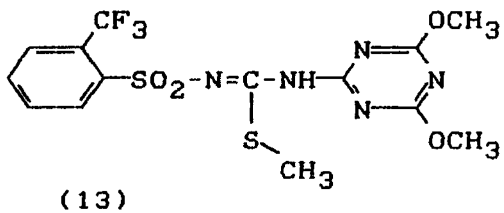 (13) | 125 | 0 | 90 | 90 | 95 | 90 |
| 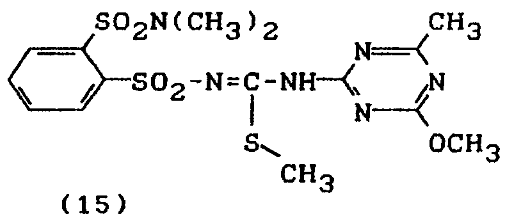 (15) | 125 | 0 | 100 | 100 | 95 | 95 |
| 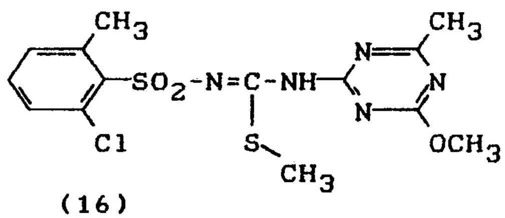 (16) | 125 | 10 | 95 | 100 | 95 | 95 |
| 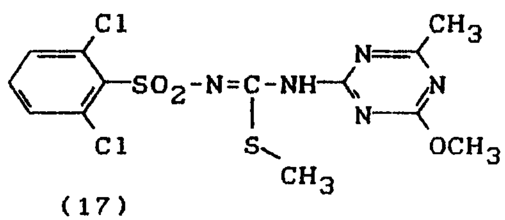 (17) | 125 | 0 | 100 | 100 | 100 | 100 |

**Tabelle B:**

| Wirkstoff (Bsp.-Nr.) | Aufwandmenge (g/ha) | Weizen | Galinsoga | Matricaria | Sinapis | Stellaria |
|---|---|---|---|---|---|---|
| 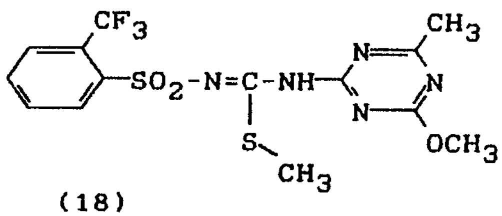 (18) | 125 | 0 | 100 | 100 | 100 | 100 |
| 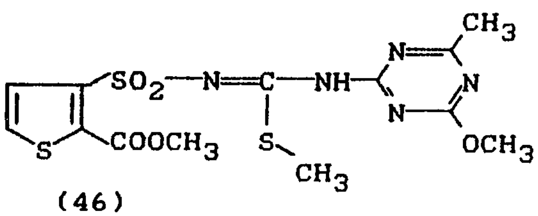 (46) | 125 | 0 | 95 | 95 | 90 | 90 |
| 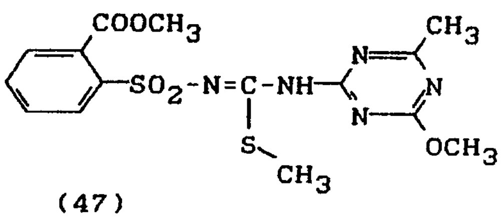 (47) | 125 | 0 | 100 | 100 | 100 | 100 |
| 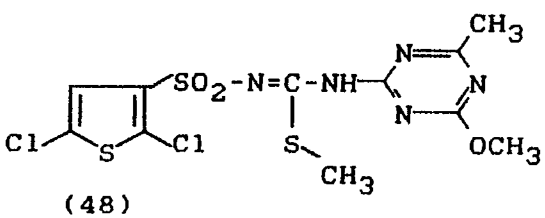 (48) | 125 | 0 | 90 | 20 | 80 | 80 |

**Tabelle B:**

| Wirkstoff (Bsp.-Nr.) | Aufwandmenge (g/ha) | Weizen | Galinsoga | Matricaria | Sinapis | Stellaria |
|---|---|---|---|---|---|---|
| 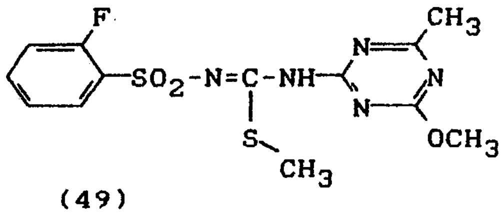 (49) | 125 | 0 | 90 | 95 | 95 | 95 |
| 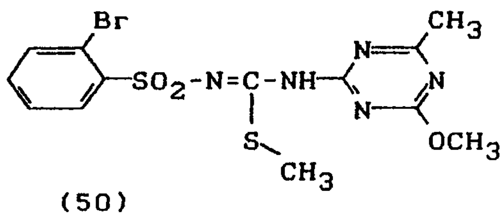 (50) | 125 | 0 | 100 | 100 | 100 | 100 |
| 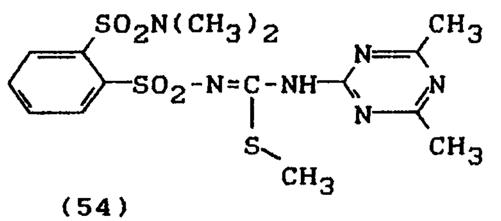 (54) | 125 | 10 | 95 | 50 | 95 | 40 |
| 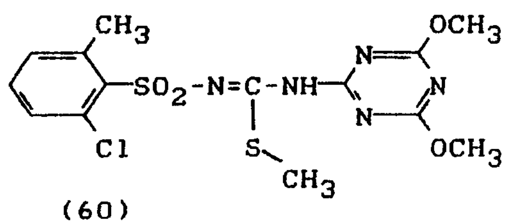 (60) | 125 | 0 | 90 | 95 | 100 | 90 |

Tabelle B:

| Wirkstoff (Bsp.-Nr.) | Aufwandmenge (g/ha) | Weizen | Galinsoga | Matricaria | Sinapis | Stellaria |
|---|---|---|---|---|---|---|
| 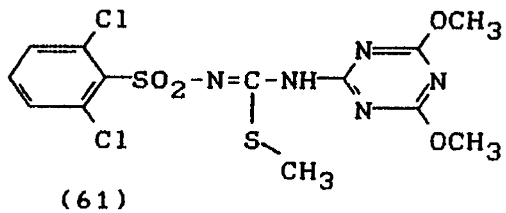 (61) | 125 | 0 | 90 | 100 | 95 | 95 |
| 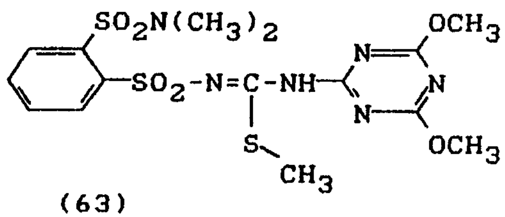 (63) | 125 | 0 | 100 | 100 | 100 | 100 |
| 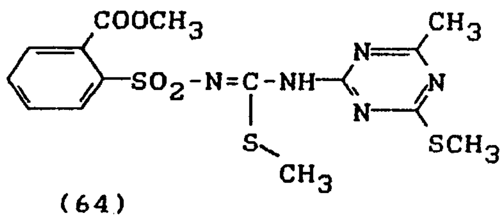 (64) | 125 | 0 | 40 | 70 | 80 | 80 |
| 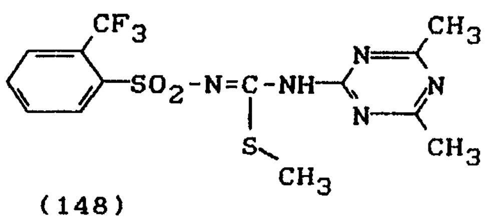 (148) | 125 | 0 | 30 | 80 | 90 | 95 |

Tabelle B:

| Wirkstoff (Bsp.-Nr.) | Aufwandmenge (g/ha) | Weizen | Galinsoga | Matricaria | Sinapis | Stellaria |
|---|---|---|---|---|---|---|
| 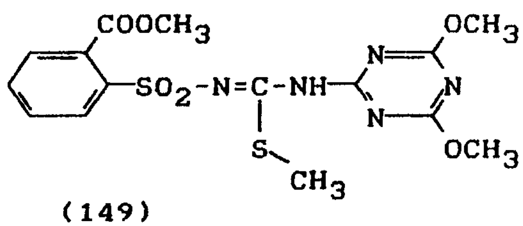 (149) | 125 | 0 | 40 | 90 | 95 | 90 |
| 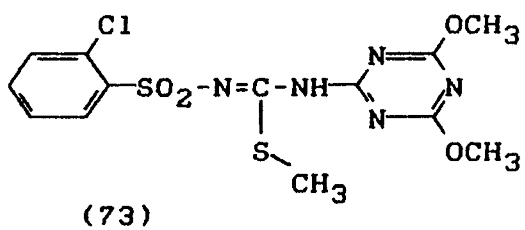 (73) | 125 | 0 | 90 | 90 | 100 | 90 |

Herstellungsbeispiele:

Herstellung der in Tabelle 1 als Bsp.-Nr. 61 aufgeführten Verbindung:

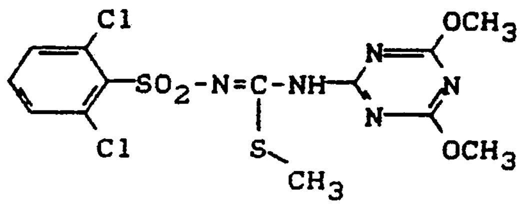

11,2 g (0,046 Mol) 2,6-Dichlor-benzolsulfonsäurechlorid werden unter Rühren zu einer auf 0° C abgekühlten Mischung aus 10,5 g (0,046 Mol) N-(4,6-Dimethoxy-s-triazin-2-yl)-S-methyl-isothioharnstoff und 74 ml Pyridin portionsweise gegeben und das Gemisch wird dann 20 Stunden bei 20° C gerührt. Anschließend wird das Reaktionsgemisch unter Rühren zu einer auf 0° C bis 5° C abgekühlten Mischung aus 90 ml konz. Salzsäure und 370 ml Wasser gegeben und das hierbei kristallin angefallene Produkt wird durch Absaugen isoliert.

Man erhält 12,3 g (62% der Theorie) N-(4,6-Dimethoxy-s-triazin-2-yl)-N′-(2,6-dichlor-phenylsulfonyl)-S-methyl-isothioharnstoff vom Schmelzpunkt 185° C.

Herstellung der in Tabelle 1 als Beispiel Nr. 152 aufgeführten Verbindung:

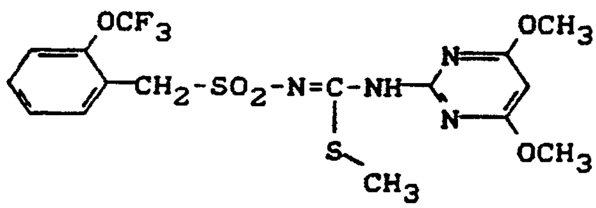

Eine Lösung aus 13,7 g (0,06 Mol) N-(4,6-Dimethoxy-pyrimidin-2-yl)-S-methyl-isothioharnstoff und 13,6 g (0,12 Mol) 1,4-Diaza-bicyclo[2,2,2]-octan (DABCO) in 150 ml Methylenchlorid wird bei 0° C unter Rühren tropfenweise mit einer Lösung aus 33 g (0,12 Mol) 2-Trifluormethoxybenzylsulfochlorid in 50 ml Methylenchlorid versetzt.

Man läßt auf Raumtemperatur kommen und rührt noch 2 Stunden nach. Der ausgefallene Kristallbrei wird abgesaugt, in Wasser verrührt und erneut abgesaugt. Nach Waschen des Rückstandes mit Ethanol/Ether (ca. 1:10) und Trocknen werden 25 g (0,054 Mol, 89% der Theorie) an N-(4,6-Dimethoxy-pyrimidin-2-yl)-N′-(2-trifluormethoxybenzylsulfonyl)-S-methyl-isothioharnstoff vom Schmelzpunkt 173° C erhalten.

Analog können auch die anderen in Tabelle 1 aufgeführten Verbindungen der Formel (I) hergestellt werden.

## Ansprüche

1. Selektiv-herbizide Mittel, gekennzeichnet durch einen Gehalt an mindestens einem Sulfonylisothioharnstoff der Formel (I),

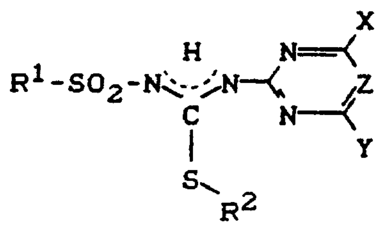

(I)

in welcher
$R^1$ für Phenyl oder Pyridyl steht, welche jeweils 1 bis 3, gleiche oder verschiedene Substituenten aus der Reihe Halogen, Cyano, $C_1$-$C_4$-Alkyl, $C_1$-$C_4$-Halogenalkyl, $C_1$-$C_4$-Alkoxy, $C_1$-$C_4$-Alkylthio, $C_1$-$C_4$-Alkylsulfinyl, $C_1$-$C_4$-Alkylsulfonyl, Di-($C_1$-$C_4$-alkyl)-amino-sulfonyl, N-($C_1$-$C_4$-Alkoxy)-N-($C_1$-$C_4$-alkyl)-aminosulfonyl, Phenyl, Phenylsulfonyl, $C_1$-$C_6$-Alkoxy-carbonyl (welches gegebenenfalls durch Halogen oder $C_1$-$C_4$-Alkoxy substituiert ist) enthalten, oder
$R^1$ für Benzyl oder Pyridylmethyl steht, welche jeweils 1 bis 3, gleiche oder verschiedene Substituenten aus der Reihe Halogen, Cyano, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkoxy-carbonyl, Di-($C_1$-$C_4$-alkyl)-amino-carbonyl enthalten, oder
$R^1$ für Thienyl oder Pyrazolyl steht, welche jeweils 1 bis 3, gleiche oder verschiedene Substituenten aus der Reihe Halogen, $C_1$-$C_4$-Alkyl, $C_1$-$C_4$-Halogenalkyl, $C_1$-$C_4$-Alkoxy-carbonyl enthalten, oder
$R^1$ für Naphthyl steht,
$R^2$ für $C_1$-$C_4$-Alkyl (welches gegebenenfalls durch Halogen, Cyano, $C_1$-$C_4$-Alkoxy, Carboxy, $C_1$-$C_4$-Alkoxy-carbonyl, Aminocarbonyl, $C_1$-$C_4$-Alkylamino-carbonyl oder Di-($C_1$-$C_4$-alkyl)-amino-carbonyl substituiert ist), für Benzyl oder für Phenylethyl steht (welche jeweils gegebenenfalls durch Halogen, $C_1$-$C_4$-Alkyl, $C_1$-$C_4$-Alkoxy oder $C_1$-$C_4$-Alkoxy-carbonyl substituiert sind),
X für Wasserstoff, Halogen, $C_1$-$C_4$-Alkyl, $C_1$-$C_4$-Halogenalkyl, Cyclopropyl, $C_1$-$C_4$-Alkoxy, $C_1$-$C_4$-Halogenalkoxy, $C_1$-$C_4$-Alkylthio, $C_1$-$C_4$-Alkylamino oder Di-($C_1$-$C_4$-alkyl)-amino steht,
Y für $C_1$-$C_4$-Alkyl, $C_1$-$C_4$-Halogenalkyl, Cyclopropyl, $C_1$-$C_4$-Alkoxy oder $C_1$-$C_4$-Halogenalkoxy steht und
Z für Stickstoff oder eine C-$R^3$-Gruppierung steht,
worin
$R^3$ für Wasserstoff, Halogen, $C_1$-$C_4$-Alkyl, $C_1$-$C_4$-Alkoxy, $C_1$-$C_4$-Halogenalkoxy oder $C_1$-$C_4$-Alkoxy-$C_1$-$C_4$-alkoxy steht.

2. Selektiv-herbizide Mittel gemäß Anspruch 1, gekennzeichnet durch einen Gehalt an einem Sulfonylisothioharnstoff der Formel (I), in welcher
$R^1$ für Phenyl oder Pyridyl, welche jeweils 1 oder 2,gleiche oder verschiedene Substituenten aus der Reihe Fluor, Chlor, Brom, Cyano, Methyl, Trifluormethyl, Methoxy, Ethoxy, Methylthio, Ethylthio, Propylthio, Isopropylthio, Methylsulfinyl, Ethylsulfinyl, Methylsulfonyl, Ethylsulfonyl, Dimethylaminosulfonyl, Diethylaminosulfonyl, N-Methoxy-N-methyl-aminosulfonyl, Phenyl, Phenylsulfonyl, Methoxycarbonyl, Ethoxycarbonyl, Propoxycarbonyl, Isopropoxycarbonyl, 2-Chlor-ethoxy-carbonyl, 2-Methoxy-ethoxy-carbonyl, 2-Ethoxyethoxy-carbonyl enthalten, oder
$R^1$ für Benzyl oder Pyridylmethyl, welche jeweils 1 oder 2, gleiche oder verschiedene Substituenten aus der Reihe Fluor, Chlor, Brom, Methyl, Trifluormethyl, Methoxy, Difluormethoxy, Trifluormethoxy, Methoxycarbonyl, Ethoxycarbonyl, Dimethylaminocarbonyl enthalten, oder
$R^1$ für Thienyl oder Pyrazolyl, welche jeweils 1 oder2, gleiche oder verschiedene Substituenten aus der Reihe Fluor, Chlor, Brom, Methyl, Ethyl, Trifluormethyl, Methoxycarbonyl, Ethoxycarbonyl enthalten, oder für Naphthyl,
$R^2$ für Methyl, Ethyl, Cyanomethyl, Carboxymethyl, Methoxycarbonylmethyl, Ethoxycarbonylmethyl oder Benzyl,
X für Wasserstoff, Chlor, Methyl, Ethyl, Trifluormethyl, Cyclopropyl, Methoxy, Ethoxy, Difluormethoxy, Methylthio, Methylamino oder Dimethylamino,
Y für Methyl, Ethyl, Trifluormethyl, Cyclopropyl, Methoxy, Ethoxy oder Difluormethoxy und
Z für Stickstoff oder eine C-$R^3$-Gruppierung steht,
worin
$R^3$ für Wasserstoff, Chlor oder Methyl steht.

3. Verfahren zur selektiven Unkrautbekämpfung in Kulturpflanzen, dadurch gekennzeichnet, daß man Sulfonylisothioharnstoffe der Formel (I) gemäß Anspruch 1 auf die Unkräuter und/oder ihren Lebensraum einwirken läßt.

4. Verwendung von Sulfonylisothioharnstoffen der Formel (I) gemäß Anspruch 1 zur selektiven Bekämpfung

von Unkräutern in Kulturpflanzen.

5. Verfahren zur Herstellung von selektiv-herbiziden Mitteln, dadurch gekennzeichnet, daß man Sulfonylisothioharnstoffe der Formel (I) gemäß Anspruch 1 mit Streckmitteln und/oder oberflächenaktiven Mitteln vermischt.

6. Neue Sulfonylisothioharnstoffe der Formel (Ia),

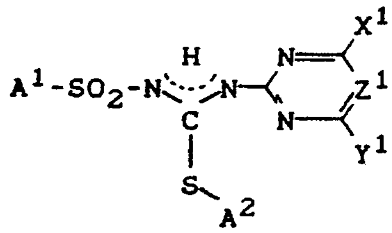

(Ia)

in welcher

$A^1$ für Benzyl, welches durch $C_1$-$C_4$-Halogenalkyl, $C_1$-$C_4$-Halogenalkoxy oder $C_1$-$C_4$-Alkoxycarbonyl substituiert ist, oder für Pyridylmethyl, welches durch Halogen, $C_1$-$C_4$-Alkyl, $C_1$-$C_4$-Halogenalkyl, $C_1$-$C_4$-Alkoxy oder $C_1$-$C_4$-Halogenalkoxy substituiert ist, steht,

$A^2$ für $C_1$-$C_4$-Alkyl (welches gegebenenfalls durch Halogen, Cyano, $C_1$-$C_4$-Alkoxy, Carboxy, $C_1$-$C_4$-Alkoxycarbonyl, Aminocarbonyl, $C_1$-$C_4$-Alkylamino-carbonyl oder Di-($C_1$-$C_4$-alkyl)-amino-carbonyl sustituiert ist), für Benzyl oder für Phenylethyl steht (welche jeweils gegebenenfalls durch Halogen, $C_1$-$C_4$-Alkyl, $C_1$-$C_4$-Alkoxy oder $C_1$-$C_4$-Alkoxy-carbonyl substituiert sind),

$X^1$ für Wasserstoff, Halogen, $C_1$-$C_4$-Alkyl, $C_1$-$C_4$-Halogenalkyl, Cyclopropyl, $C_1$-$C_4$-Alkoxy, $C_1$-$C_4$-Alkylthio, $C_1$-$C_4$-Alkylamino oder Di-($C_1$-$C_4$-alkyl)-amino steht,

$Y_1$ für $C_1$-$C_4$-Alkyl, $C_1$-$C_4$-Halogenalkyl, Cyclopropyl oder $C_1$-$C_4$-Alkoxy steht und

$Z^1$ für Stickstoff oder eine CH-Gruppierung steht.

7. Verfahren zur Herstellung von Sulfonylisothioharnstoffen der Formel (Ia) gemäß Anspruch 6, dadurch gekennzeichnet, daß man Sulfonsäurechloride der allgemeinen Formel (II),

$A^1$-$SO_2$-Cl (II)

in welcher

$A^1$ die in Anspruch 6 angegebene Bedeutung hat,

mit Isothioharnstoffen der allgemeinen Formel (III)

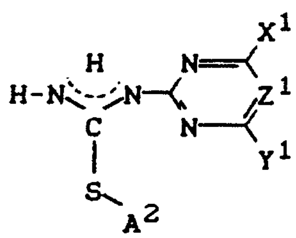

(III)

in welcher

$A^2$, $X^1$, $Y^1$ und $Z^1$ die in Anspruch 6 angegebene Bedeutung haben,

gegebenenfalls in Gegenwart von Säureakzeptoren und gegebenenfalls in Gegenwart von Verdünnungsmitteln bei Temperaturen zwischen -20° C und +30° C umsetzt.

8. Neue Sulfonylisothioharnstoffe der Formel (Ia) gemäß Anspruch 6, dadurch gekennzeichnet, daß darin

$A^1$ für Benzyl, welches in ortho-Position durch Difluormethoxy, Trifluormethoxy oder Methoxycarbonyl substituiert ist, oder für Pyridylmethyl welches durch Fluor, Chlor, Brom Methyl, Trifluormethyl oder Methoxy substituiert ist, steht,

$A^2$ für Methyl, Ethyl, Cyanomethyl, Carboxymethyl, Methoxycarbonylmethyl, Ethoxycarbonylmethyl oder Benzyl steht,

$X^1$ für Methyl, Ethyl, Trifluormethyl, Cyclopropyl, Methoxy oder Ethoxy steht,

$Y^1$ für Methyl, Ethyl, Trifluormethyl, Cyclopropyl, Methoxy oder Ethoxy steht und

$Z^1$ für Stickstoff oder eine CH-Gruppierung steht.

9. Die Verbindungen N′-(4,6-Dimethoxy-s-triazin-2-yl)-N″-(2-trifluormethyl-phenylsulfonyl)-S-methyl-isothioharnstoff, N′-(4-Methoxy-6-methyl-s-triazin-2-yl)-N″-(2-chlor-6-methyl-phenylsulfonyl)-S-methyl-isothioharnstoff, N′-(4-Methoxy-6-methyl-s-triazin-2-yl)-N″-(2,5-dichlor-3-thienylsulfonyl)-S-methyl-isothioharnstoff, N′-(4-Methoxy-6-methyl-s-triazin-2-yl )-N″-(2-fluor-phenylsulfonyl)-S-methyl-isothioharnstoff, N′-(4-Methoxy-6-methyl-s-triazin-2-yl)-N″-(2-brom-phenylsulfonyl)-S-methyl-isothioharnstoff, N′-(4,6-Dimethoxy-s-triazin-2-yl)-N″-(2-chlor-6-methyl-phenylsulfonyl)-S-methyl-isothioharnstoff, N′-(4,6-Dimethoxy-s-triazin-2-yl)-N″-(2,6-dichlor-phenylsulfonyl)-S-methyl-isothioharnstoff und N′-(4-Methyl-6-methylthio-s-triazin-2-yl)-N″-(2-methoxycarbonyl-phenylsulfonyl)-S-methyl-isothioharnstoff
gemäß Anspruch 6.